# EUROPEAN PATENT APPLICATION

(11) **EP 3 527 571 A1**
(43) Date of publication of application: **21.08.2019**
(21) Application number: 18156797.5
(22) Date of filing: 14.02.2018
(51) Int. Cl.: C07D 473/24, A61P 31/00, A61K 31/52

(54) **PYRIMIDINE DERIVATIVES FOR PREVENTION AND TREATMENT OF BACTERIAL INFECTION**

(71) Applicant: Université de Liège, 4000 Liège (BE)
(72) Inventor: LANCELLOTTI, Patrizio, 4000 LIEGE (BE); OURY, Cécile, 4000 LIEGE (BE); PIROTTE, Bernard, 4000 LIEGE (BE)
(74) Representative: Bosch, Henry

(57) **Abstract**

New pyrimidine derivatives of formula (I) for use in treatment or prevention of bacterial infection in a host mammal in need of such treatment or prevention and use as inhibitor of biofilm formation on a surface of biomaterial or medical device, particularly of cardiovascular device such as prosthetic heart valve or pacemakers.

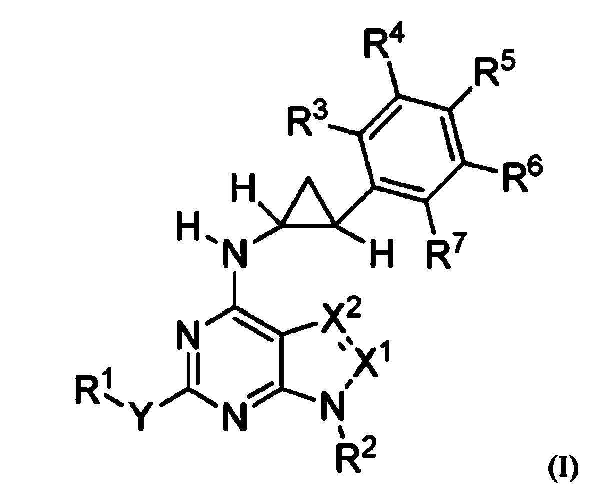

## Description

The present invention relates to new pyrimidine derivatives, compositions containing them and a method of preparation thereof. The present invention also relates to new pyrimidine derivatives for use in prevention and treatment of bacterial infection.

Bacteria are often incriminated in healthcare-associated infections (including medical device-related infections), causing increased patient morbidity and mortality, and posing huge financial burden on healthcare services. The situation has become critical since more and more bacteria are becoming resistant to antibiotics belonging to various classes such as Penicillins, Carbapenems, Cephalosporins, Quinolones, Aminoglycosides, and Glycopeptides, and an increasing number of infections are becoming difficult to cure.

The increasing resistance to antibiotics is a growing public health concern because of the limited treatment options available for these serious infections. According to the World Health Organization, « *antibiotic resistance is one of the biggest threats to global health, food security, and development today. Antibiotic resistance can affect anyone, of any age, in any country. Antibiotic resistance occurs naturally, but misuse of antibiotics in humans and animals is accelerating the process. Antibiotic resistance leads to longer hospital stays, higher medical costs and increased mortality. »*

In Europe, antibiotic resistance causes approximately 25,000 deaths per year and 2.5 millions extra hospital days (Source: Center for Disease Control and prevention, Global Health). The clinical burden associated with antimicrobial resistance is estimated to cost approximately €1.5 billion per year.
For instance, in 15 European countries more than 10% of bloodstream *Staphylococcus aureus* infections are caused by methicillin-resistant strains (MRSA), with several of these countries seeing resistance rates closer to 50% (European Centre for Disease Prevention and Control Antimicrobial Resistance Interactive Database (EARS-NET)). According to the Center for Disease Control and Prevention, there were more than 80,000 invasive MRSA infections and 11,285 related deaths in 2011. A study by researchers at UC Davis finds that the number of children hospitalized due to community-acquired MRSA doubled between 2000 and 2007 (AHRQ statistical).

MRSA is epidemic in some regions of the world.

From 2010 to 2014, the rate of *S. aureus* septicemias increased nearly 20 percent, from 2.66 to 3.15 per 1,000 hospitalizations. The rate of MRSA septicemias increased from 1.45 to 1.53 per 1,000 hospitalizations and methicillin-susceptible *S*. *aureus* (MSSA) septicemia increased from 1.21 to 1.61 per 1,000 hospitalizations (U.S. Centers for Disease Control and Prevention (CDC).

While most MSRA infections occur in hospitals, the number and severity of infections in the community appears to be increasing. Some 30 percent of people have staph bacteria on their skin (up to 12 million outpatient visits each year for skin infections).

MRSA infections are the leading cause of skin and soft tissue infections among hospital patients, and can result in severe and even fatal disease. These infections account for almost 19,000 deaths and more than 94,000 life-threatening illnesses each year in the US.

The number of infections with vancomycin-resistant enterococci (VRE) in US hospitals increased from 9,820 in 2000 to 21,352 in 2006 (Ramsey AM, Zilberberg MD. Secular trends of hospitalization with vancomycin-resistant Enterococcus infection in the United States, 2000-2006. Infect Control Hosp Epidemiol. 2009;30:184-186).

*E. faecium* is now almost as common a cause of nosocomial infections as *E. faecalis.* This change in species is of paramount clinical importance, as *E. faecium* is by far the more difficult of the two species to treat. For example, in the US, the percentage of *E. faecium* isolates that were resistant to vancomycin rose from 0 % before the mid 1980s to more than 80 % by 2007; by contrast, only ∼5 % of *E. faecalis* isolates are vancomycin resistant (Arias CA, Murray BE. Emergence and management of drug-resistant enterococcal infections. Expert Rev Anti Infect Ther. 2008;6:637-655).

By 2007, vancomycin resistance among clinical enterococcal isolates from Europe varied from >30 % in countries such as Greece and Ireland to less than 1 % in Scandinavian countries, although a recent alarming report from Sweden documented an approximately fourfold increase in infections by VRE in the period 2007-2009 compared with 2000-2006 (Soderblom T, et al. Alarming spread of vancomycin resistant enterococci in Sweden since 2007. Euro Surveill. 2010;15:19629).

The use of antibiotics is not safe especially in long-term therapy or high dose therapy. Such environmental pressure may promote selection of resistant bacteria, population, altering population structure and increasing the risk of horizontal gene transfer leading to the mobility of resistant genes into the microbiome.

Antibiotic treatment targets both the « good » and the « bad » bacteria.

The human gastro-intestinal tract (GI) microbiota is made of about trillions of microorganisms most of them bacteria. Microbiota and host's defense relationship is essential for metabolic and physiological functions contributing to health. By disrupting this benefit interaction, dietary components, physical and psychological stress, drugs but also antibiotics increase incidence of several diseases like obesity, inflammation and cardiovascular diseases (CVD). CVD remain the first cause of death in industrial society with growing incidence in other countries.

For instance recent studies showed a direct link between long term antibiotics treatment, disruption of GI microbiota and risks of atherosclerosis in mice.

The source of bacterial infection is diverse and there is a large number of bacterial infections.

Infections caused by Gram-positive bacteria represent a major public health burden, not just in terms of morbidity and mortality, but also in terms of increased expenditure on patient management and implementation of infection control measures. *Staphylococcus aureus* and *enterococci* are established pathogens in the hospital environment, and their frequent multidrug resistance complicates therapy.

*Staphylococcus aureus* is an important pathogen responsible for a broad range of clinical manifestations ranging from relatively benign skin infections to life-threatening conditions such as endocarditis and osteomyelitis. It is also a commensal bacterium (colonizing approximately 30 percent of the human population).
Two major shifts in *S*. *aureus* epidemiology have occurred since the 1990s: an epidemic of community-associated skin and soft tissue infections (largely driven by specific methicillin-resistant *S*. *aureus* [MRSA] strains), and an increase in the number of healthcare-associated infections (especially infective endocarditis and prosthetic device infections).

The emergence of Glycopeptide resistance in *Staphylococcus aureus* (GISA) is another source of great concern because, especially in hospitals, this class of antibiotics, particularly vancomycin, is one of the main resources for combating infections caused by methicillin-resistant Staphylococcus aureus strains (MRSA). Although the prevalence of GISA is relativley low (accounting for approximately 1.3% of all MRSA isolates tested), mortality due to GISA infections is very high (about 70%), especially among patients hospitalised in high-risk departments, such as intensive care units (ICU).

Coagulase-negative staphylococci (CoNS) are the most frequent constituent of the normal flora of the skin. These organisms are common contaminants in clinical specimens as well as increasingly recognized as agents of clinically significant infection, including bacteremia and endocarditis. Patients at particular risk for CoNS infection include those with prosthetic devices, pacemakers, intravascular catheters, and immunocompromised hosts.
Coagulase-negative staphylococci account for approximately one-third of bloodstream isolates in intensive care units, making these organisms the most common cause of nosocomial bloodstream infection.
Enterococcal species can cause a variety of infections, including urinary tract infections, bacteremia, endocarditis, and meningitis. Enterococci are relatively resistant to the killing effects of cell wall-active agents (penicillin, ampicillin, and vancomycin) and are impermeable to aminoglycosides.
Vancomycin-resistant enterococci (VRE) are an increasingly common and difficult-to-treat cause of hospital-acquired infection.
Multiple epidemics of VRE infection have been described in diverse hospital settings (e.g., medical and surgical intensive care units, and medical and pediatric wards) and, like methicillin-resistant *Staphylococcus aureus,* VRE is endemic in many large hospitals.
The beta-hemolytic *Streptococcus agalactiae* (Group B Streptococcus, GBS) is another Gram-positive bacteria. The bacteria can cause sepsis and/or meningitis in the newborn infants. It is also an important cause of morbidity and mortality in the elderly and in immuno-compromised adults. Complications of infection include sepsis, pneumonia, osteomyelitis, endocarditis, and urinary tract infections.
Importantly, methicillin-resistant *S*. *aureus* (MRSA), and vancomycin-resistant enterococci (VRE) are in the WHO list of antibiotic-resistant "high priority pathogens" that pose the greatest threat to human health (February 2017). "This list is a new tool to ensure R&D responds to urgent public health needs." High priority refers to high urgency of need for new antibiotics.
Besides human medicine, actions need also to be taken in veterinary medicine. Indeed, companion animals, such as cats, dogs, and horses, can be colonized and infected by MRSA, without host adaptation, and therefore may act as reservoirs for human infections. Bacteria can also develop distinct resistance when hosted by animals. Thus, different antibiotic usage patterns between human and veterinary medicine can shape the population of a major human pathogen, highlighting the importance of the « one health » concept to implement antibacterial approaches.

The factors that make these bacteria especially adept at surviving on various biomaterials include adherence and production of biofilm (see below).
The four above mentioned bacteria have the ability to form biofilms on any surface biotic and abiotic. The initial step of biofilm formation is the attachment/adherence to surface, which is stronger in shear stress conditions. The protein mainly responsible for this adhesion is the polysaccharide intercellular adhesin (PIA), which allows bacteria to bind to each other, as well as to surfaces, creating the biofilm. The second stage of biofilm formation is the development of a community structure and ecosystem, which gives rise to the mature biofilm. The final stage is the detachment from the surface with consequent spreading into other locations. In all the phases of biofilm formation the quorum sensing (QS) system, mediating cell-to-cell communication, is involved.

Bacteria in the biofilm produce extracellular polymeric substances (EPS) consisting mainly of polysaccharides, nucleic acids (extracellular DNA) and proteins, that protect them from external threats, including immune system components and antimicrobials. Moreover, bacteria in the biofilm have a decreased metabolism, making them less susceptible to antibiotics; this is due to the fact that most antimicrobials require a certain degree of cellular activity in order to be effective. Another factor reinforcing such resistance is the impaired diffusion of the antibiotics throughout the biofilm because of the presence of the EPS matrix barrier.

It was also reported that in the biofilm there is higher rate of plasmid exchange increasing the chances of developing naturally occurring and antimicrobial-induced resistance.

Strategies that have been developed to eliminate biofilms target 3 different steps in the biofilm formation: inhibition of the initial stage, *i.e.* the adhesion of bacteria to surfaces; disrupting the biofilm architecture during the maturation process or step 2; inhibiting the QS system or step 3.

Because of the high resistance of these biofilms to antibiotics there is an increasing need of control and prevention of microbial growth and biofilm formation at step 2. The treatment in case of infected medical device is either a conservative treatment or the removal of the device together with a long treatment with antibiotics, but these approaches have high failure rates and elevated economical burden.

This is the reason why clinicians try to adopt a preventive approach by administering antibiotics before implantation. Another solution could be the modification of the medical devices, *e.g*. surfaces coated with silver, which have antimicrobial property or with hydrogels as well as polyurethanes, which reduce bacterial adhesion, to mention few examples.

According to Eggiman in American Society for Microbiology Press, Washington, DC 2000. p.247, pacemakers and implantable cardioverter-defibrillators [ICDs]) can become infected, with a rate of infections ranging from 0.8 to 5.7 percent.

The infection can involve subcutaneous pocket containing the device or the subcutaneous segment of the leads. Deeper infection can also occur that involves the transvenous portion of the lead, usually with associated bacteremia and/or endovascular infection.
The device and/or pocket itself can be the source of infection, usually due to contamination at the time of implantation, or can be secondary to bacteremia from a different source.

Perioperative contamination of the pacemaker pocket with skin flora appears to be the most common source of subcutaneous infection.

Cardiac device-related infective endocarditis (CDRIE) is another life-threatening condition, with increasing incidence due to growing number of implantations (81,000 pacemaker implantation per year in Europe). The incidence of CDRIE reaches 0.14 percent, and is even higher after ICD implantation.

*Staphylococcus aureus* and coagulase-negative staphylococci (often *Staphylococcus epidermidis*) cause 65 to 75 percent of generator pocket infections and up to 89 percent of device-related endocarditis. Episodes arising within two weeks of implantation are more likely to be due to *S*. *aureus.*

Successful treatment of an infected medical device or biomaterial, regardless of the involved component, generally requires removal of the entire system and administration of antibiotics targeting the causative bacteria. Importantly, medical therapy alone is associated with high mortality and risk of recurrence.

Prosthetic valve endocarditis (PVE) is a serious infection with potentially fatal consequences.

Bacteria can reach the valve prosthesis by direct contamination intraoperatively or via hematogenous spread during the initial days and weeks after surgery. The bacteria have direct access to the prosthesis-annulus interface and to perivalvular tissue along suture pathways because the valve sewing ring, cardiac annulus, and anchoring sutures are not endothelialized early after valve implantation. These structures are coated with host proteins, such as fibronectin and fibrinogen, to which macroorganisms can adhere and initiate infection.
The risk of developing prosthetic valve endocarditis (PVE) is greatest during the initial three months after surgery, remains high through the sixth month, and then falls gradually with an annual rate of approximately 0.4 percent from 12 months postoperatively onward. The percentage of patients developing PVE during the initial year after valve replacement ranges from 1 to 3 percent in studies with active follow-up; by five years, the cumulative percentage ranges from 3 to 6 percent.

The most frequently encountered pathogens in early PVE (within two months of implantation) are *S*. *aureus* and coagulase-negative staphylococci.
The most frequently encountered pathogens in late PVE (two months after valve implantation) are streptococci and *S*. *aureus,* followed by coagulase-negative staphylococci and enterococci.

The coagulase-negative staphylococci causing PVE during the initial year after surgery are almost exclusively *Staphylococcus epidermidis.* Between 84 and 87 percent of these organisms are methicillin resistant and thus resistant to all of the beta-lactam antibiotics.

According to the 2008 French survey, PVE accounts for about 20 percent of all infective endocarditis. PVE is related to health care in about 30 percent of cases. *S*. *aureus* is the first causative pathogen, being responsible for more than 20 percent of PVE. Importantly, when comparing data from 1999, PVE-related mortality remains high, reaching about 40 percent after surgery, and 25 percent in-hospital mortality.

Periprosthetic joint infection (PJI) occurs in 1 to 2 percent of joint replacement surgeries and is a leading cause of arthroplasty failure.

Biofilms play an important role in the pathogenesis of PJIs. Bacteria within biofilm become resistant to therapy; as a result, antibacterial therapy is often unsuccessful unless the biofilm is physically disrupted or removed by surgical debridement.
Prosthetic joint infections are categorized according to the timing of symptom onset after implantation: early onset (<3 months after surgery), delayed onset (from 3 to 12 months after surgery), and late onset (>12 months after surgery). These infections have the following characteristics. Early-onset infections are usually acquired during implantation and are often due to virulent organisms, such as *Staphylococcus aureus,* or mixed infections. Delayed-onset infections are also usually acquired during implantation. Consistent with the indolent presentation, delayed infections are usually caused by less virulent bacteria, such as coagulase-negative staphylococci or enterococci. Late-onset infections resulting from hematogenous seeding are typically acute and often due to *S*. *aureus,* or beta hemolytic streptococci.

The management of PJIs generally consists of both surgery and antibacterial therapy.

There is therefore an urgent need in the art for a new antibacterial therapy.

### SUMMARY OF THE INVENTION

We have surprisingly found new pyrimidine derivatives that possess antibacterial activity and can be used in the treatment or prevention of bacterial infection in a host mammal.

We have also found that such pyrimidine derivatives can be used in a method for controlling bacterial growth in biofilm formation at early stage such as step 1 or 2 or for killing bacteria at all steps of biofilm formation including the latest step 3 wherein the biofilm has reached its maturation stage of matrix formation and start detachment from the surface with a consequent spreading of bacteria into other locations.

In a first aspect, the invention provides new pyrimidine derivatives represented by formula (I) 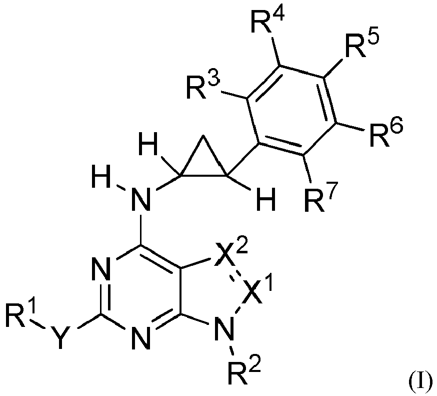 or an acceptable salt or prodrug thereof;
wherein:
X¹ and X² are independently N, CH, CR⁸ wherein R⁸ is C₁₋₆ alkyl, C₂₋₆ alkenyl or C₂₋₆ alkynyl ; with the exception that if X¹ or X² is equal to N, then X¹ and X² are different. -Y- is -O- or -S-;
R¹ and R² are independently C₁₋₆-alkyl, C₂₋₆-alkenyl, C₂₋₆-alkynyl, C₃₋₆-cycloalkyl, aryl, aryl-C₁₋₆-alkyl wherein the alkyl moiety is optionally mono or polysubstituted with OH or an halogen and the aryl moiety is optionally mono or polysubstituted with an halogen, -C₁₋₆alkyl, -C₁₋₆alkoxy, -OH, -NO₂, -CN, -NH₂, -NHR⁸, -N(R⁸)₂ -COOH, -COOR⁸,-CONH₂, -CONHR⁸, -CON(R⁸)₂, -SO₂NH₂, -SO₂NHR⁸, or -SO₂N(R⁸)₂;
R³, R⁴, R⁵, R⁶ and R⁷ are independently H, an halogen, a C₁₋₆alkyl, C₁₋₆alkoxy, -OH, -NO₂, -CN, -NH₂, -NHR⁸, -N(R⁸)₂-COOH, -COOR⁸, -CONH₂, -CONHR⁸, -CON(R⁸)₂, -SO₂NH₂,-SO₂NHR⁸, or -SO₂N(R⁸)₂;

Within its scope the invention includes all optical isomers of pyrimidine derivatives of formula (I), some of which are optically active, and also their mixtures including racemic mixtures thereof.

The term "C₁₋₆-alkyl" as used herein, alone or in combination, refers to a straight or branched, saturated hydrocarbon chain having 1 to 6 carbon atoms such as for example methyl, ethyl, n-propyl, isopropyl, n-butyl, sec-butyl, isobutyl, tert-butyl, n-pentyl, 2-methylbutyl, 3-methylbutyl, n-hexyl, 4-methylpentyl, neopentyl, n-hexyl, 1,2-dimethylpropyl, 2,2-dimethylpropyl, 1,2,2-trimethylpropyl, and the like.

The term "C₂₋₆-alkenyl" as used herein, alone or in combination, refers to a straight or branched, unsaturated hydrocarbon chain having 2 to 6 carbon atoms with at least one carbon-carbon double bond such as for example vinyl, allyl, 1-butenyl, 2-butenyl, isobutenyl, 1-pentenyl, 2-pentenyl, 3-methyl-1-butenyl, 1-methyl-2butenyl, 2,3-dimethyl-2-butenyl, 1-hexenyl, 2-hexenyl, 3-hexenyl and the like. In formula (I), the carbon-carbon double bond of C₂₋₆-alkenyl is not directly linked to the nitrogen atom, the sulfur atom or the oxygen atom.

The term "C₂₋₆-alkynyl" as used herein, alone or in combination, refers to a straight or branched, unsaturated hydrocarbon chain having 2 to 6 carbon atoms with at least one carbon-carbon triple bond such as for example acetylenyl, propenyl, 1-butynyl, 2-butynyl, 1-pentynyl, 2-pentynyl, 3-methyl-1-butynyl, 1-hexynyl, 2-hexynyl, 3-hexenyl and the like. In formula (I), the carbon-carbon triple bond of C₂₋₆-alkynyl is not directly linked to the nitrogen atom, the sulfur atom or the oxygen atom.

The term "C₃₋₆-cycloalkyl" as used herein, alone or in combination refers to a radical of a saturated cyclic hydrocarbon with 3 to 6 carbon atoms such as cyclopropyl, cyclobutyl, cyclopentyl or cyclohexyl.

The term "aryl" as used herein, alone or in combination refers to a monocyclic or polycyclic aromatic ring such as phenyl, anthracenyl, naphthyl and the like.

The term "C₁₋₆-alkoxy" as used herein, refers to a straight or branched monovalent substituent comprising a C₁₋₆-alkyl group linked through an ether oxygen having its free valence bond from the ether oxygen and having 1 to 6 carbon atoms e.g. methoxy, ethoxy, propoxy, isopropoxy, butoxy, pentoxy.

The term "-CN" as used herein refers to a carbon-nitrogen triple bond

The term halogen as used herein refers to fluoro, chloro, bromo or iodo.

The acceptable salts include pharmaceutically acceptable acid addition salts, pharmaceutically acceptable metal salts, or optionally alkylated ammonium salts, such as hydrochloric, hydrobromic, hydroiodic, phosphoric, sulfuric, trifluoroacetic, trichloroacetic, oxalic, maleic, pyruvic, malonic, succinic, citric, tartaric, fumaric, mandelic, benzoic, cinnamic, methanesulfonic, ethanesulfonic, picric acid and the like, and include acids related to the pharmaceutically acceptable salts listed in Journal of Pharmaceutical Science, 66, 2 (1977) and incorporated herein by reference, or lithium, sodium, potassium, magnesium and the like.

The term prodrug has been defined in Burger's Medicinal Chemistry and Drug discovery (5th edition 1995) as compounds, which undergo biotransformation prior to exhibiting their pharmacological effects. The term prodrug as used herein refers therefore to an analogue or a derivative of a compound of general formula (I) that comprises biohydrolysable moieties such as biohydrolysable ester functions, biohydrolysable carbamate functions, biohydrolysable ureides and the like obtained under biological conditions. Prodrugs can be prepared using well-known methods such as those described in Burgers medicinal chemistry and drug discovery (1995)172-178, 949-982 (Manfred E.Wolff).

In one embodiment, the invention relates to pyrimidine derivatives comprising a purine group and are represented by formula (I) wherein X¹ is CH or CR⁸ and X² is N; or an acceptable salt or prodrug thereof;

Preferred pyrimidine derivatives comprising a purine group are substituted by a 3,4difluorophenylcyclopropyl group as illustrated in formula II

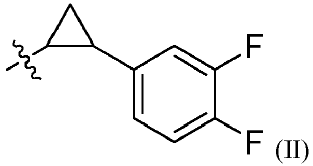

Most preferred pyrimidine derivatives comprising a purine group are substituted by a 3,4-difluorophenylcyclopropylamino group as illustrated in formula (III)

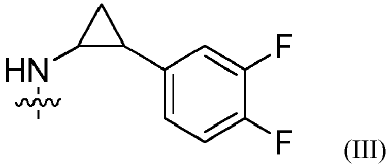

The most preferred pyrimidine derivatives comprising a purine group are:
*N*-((1*R*,2*S*)-2-(3,4-difluorophenyl)cyclopropyl)-9-methyl-2-(propylthio)-9*H*-purin-6-amine;
9-allyl-*N*-((1*R*,2*S*)-2-(3,4-difluorophenyl)cyclopropyl)-2-(propylthio)-9*H*-purin-6-amine; *N*-((1*R*,2*S*)-2-(3,4-difluorophenyl)cyclopropyl)-9-(prop-2-yn-1-yl)-2-(propylthio)-9*H-*purin-6-amine;
(1*S*,2*R*,3*S*,4*R*)-4-(6-(((1*R*,2*S*)-2-(3,4-difluorophenyl)cyclopropyl)amino)-2-(propylthio)-9*H*-purin-9-yl)cyclopentane-1,2,3-triol;
*N*-((1*R*,2*S*)-2-(3,4-difluorophenyl)cyclopropyl)-9-(prop-2-yn-1-yl)-2-(propylthio)-9*H-*purin-6-amine hydrochloride;

In another embodiment, the invention relates to pyrimidine derivatives comprising a pyrazolo group and are represented by formula (I) wherein X¹ is N and X² is CH or CR⁸ ; or an acceptable salt or prodrug thereof;
Preferred pyrimidine derivatives comprising a pyrazolo group are substituted by a 3,4difluorophenylcyclopropyl group, most preferred pyrimidine derivatives comprising a pyrazolo group are substituted by a 3,4-difluorophenylcyclopropylamino group

In another embodiment, the invention relates to pyrimidine derivatives comprising a pyrrolo group and are represented by formula (I) wherein X¹ and X² are CH or CR⁸ or an acceptable salt or prodrug thereof;

Preferred pyrimidine derivatives comprising a pyrrolo group are substituted by a 3,4-difluorophenylcyclopropylamino group. Most preferred pyrimidine derivatives comprising a pyrrolo group are substituted by 3,4-difluorophenylcyclopropylamino group.

The pyrimidine derivatives and acceptable salts or prodrugs thereof are prepared according to the following chemical pathways:
In the first embodiment, the pyrimidine derivatives according to formula (I) or
acceptable salts or prodrugs thereof, and comprising a purine group are made according to a general common chemical pathway that comprises first: a preparation of a starting product, a 2-substituted-4,6-dichloropyrimidin-5-amine Xh according to scheme 1, and further reacting the starting product with reagents according to the following steps i to iv in scheme 2. The general chemical pathway is common for purine derivatives having Y equal to -S- or -O-. Formation of 3 intermediates **Xa, Xb, Xc** wherein R¹, R², R³, R⁴, R⁵, R⁶ and R⁷ as defined in formula (I), are successively provided along the 3 steps i to iii.

Finaly, pyrimidine derivatives comprising a purine group and having Y= -O- are differentiated by an additional chemical pathway that allows conversion of the thioether (Y= S) pyrimidine derivative into a corresponding ether (Y=O) pyrimidine derivative (scheme 3).

### 1°Preparation of the starting product: 2-substituted 4,6-dichloropyrimidin-5-amines according to the following chemical pathway:

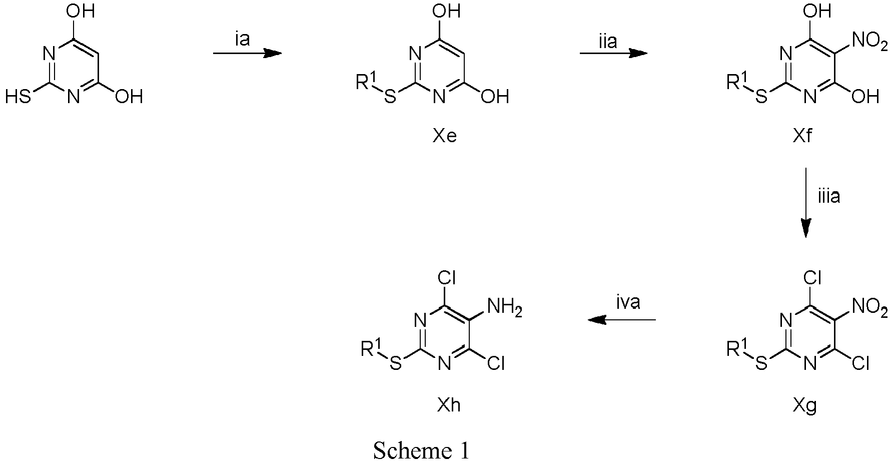

wherein R¹ is defined as above in formula (I)
A 2-substituted 4,6-dichloro-5-nitropyrimidine **Xg** was obtained by reacting thiobarbituric acid with the halide R¹X in aqueous alkaline medium such as KOH (step ia) at a temperature between 20°C to 100°C, followed by a nitration reaction using acidic medium such as nitric acid with CH₃COOH at low temperature varying from - 20°C to room temperature (step iia) and an aromatic nucleophilic substitution at a varying temperature from -20°C to 100°C using an organic base with phosphoryl chloride (step iiia)
The 2-substituted nitropyrimidine **Xg** was then reduced at room temperature by iron in acidic medium such as for example CH₃COOH to obtain the corresponding 2-substituted 4,6-dichloropyrimidin-5-amine **Xh** (step iva).

### 2° reaction of starting product with reagents according to the following steps i to iv

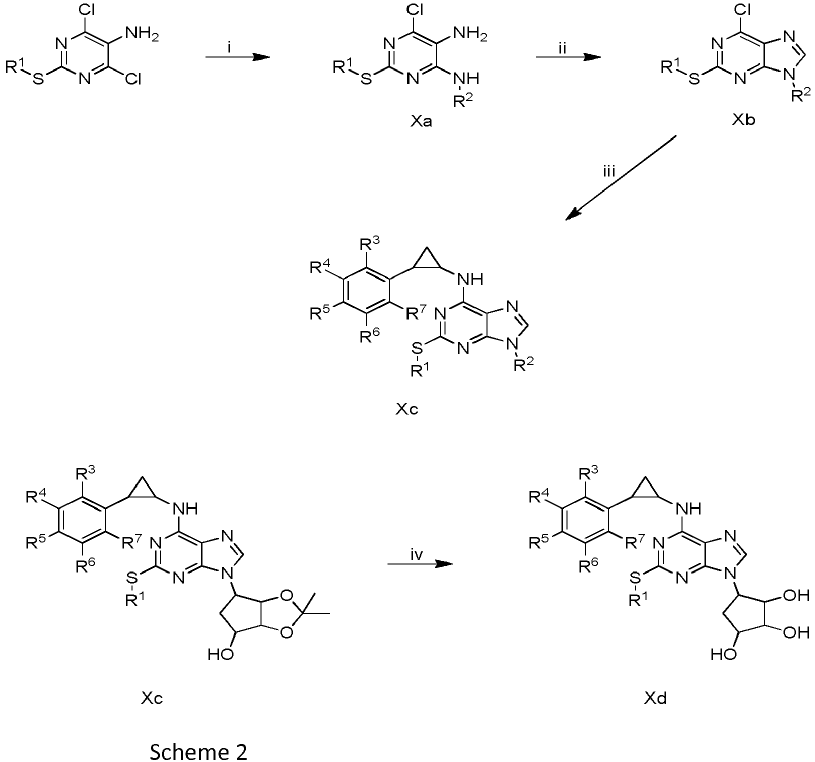

In a first step(i), *N⁴*,2-disubstituted 6-chloropyrimidine-4,5-diamines (**Xa**) are obtained by reaction of R²NH₂ with the substituted 4,6-dichloropyrimidin-5-amine Xh carried out in an alcohol such as methanol at a temperature of for example 100°C.
The first step is followed by a ring closure reaction of the intermediate **Xa** by means of triethyl orthoformate carried out at a temperature of for example 130°C, in the presence of an acid such as for example acetic acid (step ii) to obtain the corresponding intermediates 2,9- disubstituted 6-chloro-9*H*-purines (**Xb**).
In step iii, 2,9- disubstituted *N*-[(R³-R⁷)-substituted pheny]cyclopropyl-9*H*-purin-6-amines (**Xc**) are obtained by nucleophilic substitution of the chlorine atom of intermediate **Xb** by a (R³-R⁷)-substituted phenylcyclopropylamine at a temperature of for example 90°C?
In case of an acetonide of a pentane-1,2,3-triol intermediate, deprotection occured in acidic hydroalcoholic conditions to provide a pentane-1,2,3-triol final**Xd** (step iv).The reaction is carried out at a room temperature.

### 3° conversion of the thioether (Y = S) pyrimidine derivative into a corresponding ether (Y = O) pyrimidine derivative:

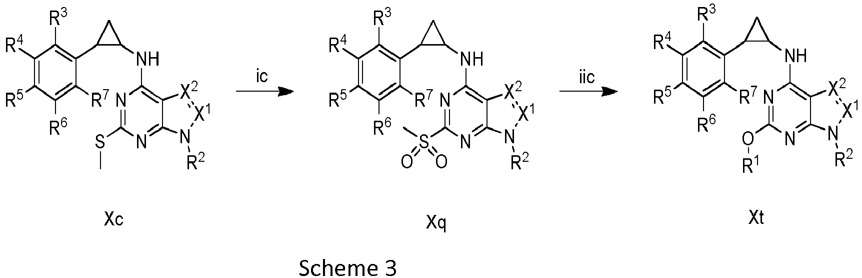

Compounds **Xt, Xu** and **Xv** that correspond to pyrimidine derivatives comprising respectively a purine, a pyrazolo and a pyrrolo group, may be obtained in two steps starting from the corresponding methylthio compound **Xc, Xk** and **Xp** (scheme3).
The first reaction consists in the oxidation of the sulfur atom of this thioether function, resulting in a methylsulfonyl group (step ic) carried out at room temperature or under heating. The substitution of **Xq, Xr** and **Xs** with an alcoolate led to the ether derivatives **Xt, Xu** and **Xv** (step iic) carried out at a temperature between 10°C and O°C.

In the second embodiment, the preparation of the pyrimidine derivatives comprising a pyrazolo group and represented by formula (I) wherein X¹ is N and X² is CH or CR⁸ ; or an acceptable salt or prodrug thereof are generally made according to the following chemical pathway: 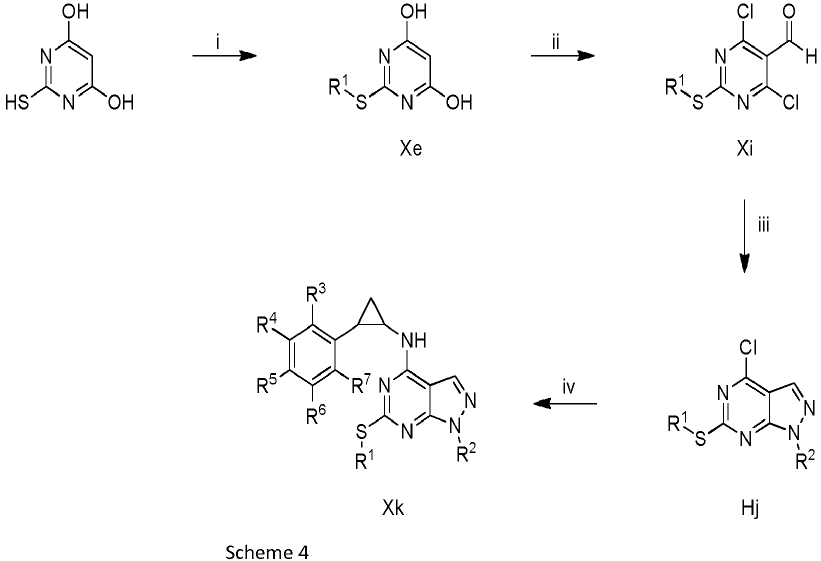 wherein R¹, R², R³, R⁴, R⁵, R⁶ and R⁷ are defined in the general formula (I).
After reaction between thiobarbituric acid and R¹X halide at a temperature of for example 80°C (step i), the 2-substituted pyrimidine 4,6-diol **Xe** is reacted with phosphoryl chloride in presence of DMF at a temperature between 0°C and 110°C to obtain the corresponding 2- substituted 4,6-dichloropyrimidine-5-carbaldehyde **Xi** (step ii).
The ring closure reaction of **Xi** by means of a substituted hydrazine provided the corresponding 1*H*-pyrazolo[3,4-*d*]pyrimidine **Hj** at a temperature between -80°C and 0°C (step iii).
Further conversion of the thioether (Y = S) pyrimidine derivative into a corresponding ether(Y=O) pyrimidine derivative (**Xu**) can be made according to scheme 3 as described above.

In the third embodiment, the preparation of the pyrimidine derivatives comprising a pyrrolo group and represented by formula (I) wherein X¹ and X² are CH or CR^{8;;} or an acceptable salt or prodrug thereof are generally made according to the following chemical pathway: 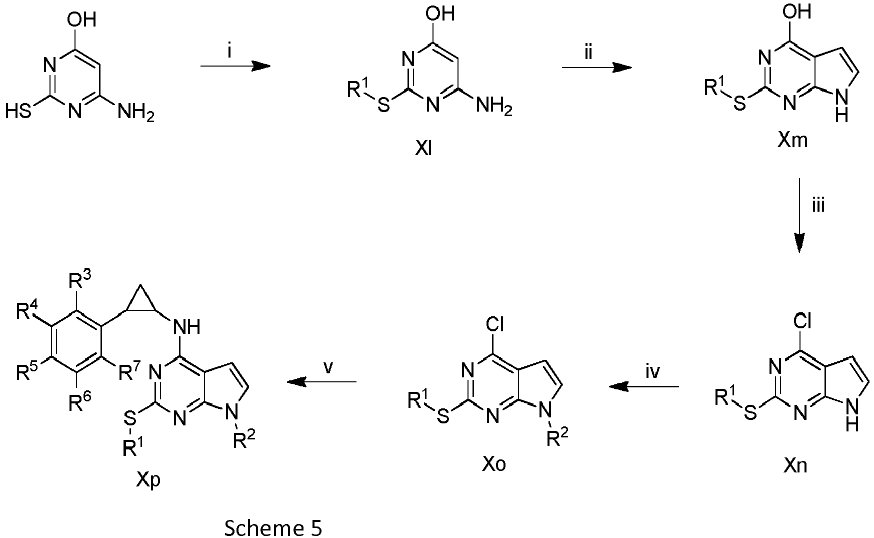 wherein R¹, R², R³, R⁴, R⁵, R⁶ and R⁷ are defined as defined in formula (I).
The 2-substituted 6-chloro-4-aminopyrimidine **Xl** was obtained by reacting 4-amino-6-chloropyrimidine-2-thiol with the R¹X halide in alcaline medium a temperature between 70°C and 110°C (step i).
**Xl** is converted into the corresponding 7*H*-pyrrolo[2,3-*d*]pyrimidine **Xm** by means of chloroacetaldehyde at a temperature between 60°C and 100°C (step ii).
An aromatic nucleophilic substitution using phosphoryl chloride at a temperature between 0°C and 110°C is then achieved to give **Xn** (step iii), followed by a N-alkylation to give **Xo** (step iv) at a temperature between 0°C and 100°C.
2,7-disubstituted *N*-[(R³-R⁷)-substituted pheny]cyclopropyl-7*H*-pyrrolo[2,3-*d*]pyrimidin-4-amines (**Xp**) were obtained by nucleophilic substitution of the chlorine atom of **Xo** by the appropriate phenylcyclopropylamine a temperature between 40°C and 90°C (step v).

Further conversion of the thioether (Y = S) pyrimidine derivative into a corresponding ether(Y=O) pyrimidine derivative (**Xv**) can be made according to scheme 3 describedabove

In a second aspect, the invention provides pyrimidine derivatives for use in the treatment or prevention of bacterial infection in a host mammal in need of such treatment.

By bacterial infection one means particularly Gram-positive bacterial infection such as for example pneumonia, septicemia, endocarditis, osteomyelitis, meningitis, urinary tract, skin, and soft tissue infections. The source of bacterial infection is diverse, and can be caused for example by the use of implantable biomaterials.

By biomaterials, one means all implantable foreign material for clinical use in host mammals such as for prosthetic joints, pacemakers, implantable cardioverter-defibrillators , intravascular catheters, coronary stent, prosthetic heart valves, intraocular lens, dental implants and the like.

By host mammal, one means preferably a human, but also an animal in need of treatment or prevention of bacterial treatment.

By prevention of bacterial infection, one means a reduction in risk of acquiring infection, or reduction or inhibition of recurrence of infection. For example, the pyrimidine derivatives may be administered as prevention before a surgical treatment to prevent infection .

In the first embodiment pyrimidine derivatives for use in the treatment or prevention of bacterial infection are pyrimidine derivatives comprising a purine group wherein X¹ is CH or CR⁸ and X² is N in formula I .

We have surprisingly found that said pyrimidine derivatives comprising a purine group with the presence of a difluorophenylcyclopropyl group exhibit antibiotic activity.

In the second embodiment pyrimidine derivatives for use in the treatment or prevention of bacterial infection are pyrimidine derivatives comprising a pyrazolo group wherein X¹ is N and X² is CH or CR⁸ in formula I .

Again said pyrimidine derivatives comprising a pyrazolo group with the presence of a difluorophenylcyclopropyl group exhibit also antibiotic activity.

In the third embodiment pyrimidine derivatives for use in the treatment or prevention of bacterial infection are pyrimidine derivatives comprising a pyrrolo group wherein X¹ and X^{2a} are CH or CR⁸ in formula I.

Again said pyrimidine derivatives comprising a pyrrolo group with the presence of a difluorophenylcyclopropyl group, also exhibit antibiotic activity.

According to the invention the pyrimidine derivative has to be administered to the patient over several days (especially in case of prevention). The pyrimidine derivatives may be administered on their own or as a pharmaceutical composition, with non-toxic doses being inferior to 3 g per day.

A further preferred aspect of the invention is a pharmaceutical composition of pyrimidine derivative for use in the prevention or treatment of bacterial infection,

The pharmaceutical composition may be a dry powder or a liquid composition having physiological compatibility. The compositions include, in addition to pyrimidine derivative, auxiliary substances, preservatives, solvents and/or viscosity modulating agents. By solvent, one means for example water, saline or any other physiological solution, ethanol, glycerol, oil such as vegetable oil or a mixture thereof. By viscosity modulating agent on means for example carboxymethylcellulose.

The pyrimidine derivative of the present invention exhibits its effects through oral, intravenous, intravascular, intramuscular, parenteral, or topical administration, and can be additionally used into a composition for parenteral administration, particularly an injection composition or in a composition for topical administration. It can also be loaded in nanoparticles for nanomedicine applications. It can be used in an aerosol composition. Such aerosol composition is for example a solution, a suspension, a micronised powder mixture and the like. The composition is administered by using a nebulizer, a metered dose inhaler or a dry powder inhaler or any device designed for such an administration.

Examples of galenic compositions include tablets, capsules, powders, pills, syrups, chewing, granules, and the like. These may be produced through well known technique and with use of typical additives such as excipients, lubricants, and binders.
Suitable auxiliary substances and pharmaceutical compositions are described in Remington's Pharmaceutical Sciences, 16th ed., 1980, Mack Publishing Co., edited by Oslo et al. Typically, an appropriate amount of a pharmaceutically-acceptable salt is used in the composition to render the composition isotonic. Examples of pharmaceutically acceptable substances include saline, Ringer's solution and dextrose solution. pH of the solution is preferably from about 5 to about 8, and more preferably from about 7 to about 7.5.

A still further aspect of the invention is a method of treatment or prevention of bacterial infection in a host mammal in need of such treatment which comprises administering to the host an effective amount of pyrimidine derivatives as defined in formula (I),
preferably pyrimidine derivatives that are substituted with a
difluorophenylcyclopropyl group;
and most preferably pyrimidine derivatives selected from the group :*N*-((1*R*,2*S*)-2-(3,4-difluorophenyl)cyclopropyl)-9-methyl-2-(propylthio)-9*H*-purin-6-amine;
9-allyl-*N*-((1*R*,2*S*)-2-(3,4-difluorophenyl)cyclopropyl)-2-(propylthio)-9*H*-purin-6-amine; *N*-((1*R*,2*S*)-2-(3,4-difluorophenyl)cyclopropyl)-9-(prop-2-yn-1-yl)-2-(propylthio)-9*H-*purin-6-amine;
(1*S*,2*R*,3*S,*4*R*)-4-(6-(((1*R*,2*S*)-2-(3,4-difluorophenyl)cyclopropyl)amino)-2-(propylthio)-9*H*-purin-9-yl)cyclopentane-1,2,3-triol;
*N*-((1*R*,2*S*)-2-(3,4-difluorophenyl)cyclopropyl)-9-(prop-2-yn-1-yl)-2-(propylthio)-9*H-*purin-6-amine hydrochloride;
or a salt or prodrug thereof.

In a last aspect, the invention provides the use of pyrimidine derivatives of formula (I) and salts or prodrugs thereof, preferably pyrimidine derivatives of formula (I) with difluorophenylcyclopropyl group;
and most preferably (*N*-((1*R*,2*S*)-2-(3,4-difluorophenyl)cyclopropyl)-9-methyl-2-(propylthio)-9*H*-purin-6-amine;
9-allyl-*N*-((1*R*,2*S*)-2-(3,4-difluorophenyl)cyclopropyl)-2-(propylthio)-9*H*-purin-6-amine; *N*-((1*R*,2*S*)-2-(3,4-difluorophenyl)cyclopropyl)-9-(prop-2-yn-1-yl)-2-(propylthio)-9*H-*purin-6-amine;
(1*S*,2*R*,3*S*,4*R*)-4-(6-(((1*R*,2*S*)-2-(3,4-difluorophenyl)cyclopropyl)amino)-2-(propylthio)-9*H*-purin-9-yl)cyclopentane-1,2,3-triol; or
*N*-((1*R*,2*S*)-2-(3,4-difluorophenyl)cyclopropyl)-9-(prop-2-yn-1-yl)-2-(propylthio)-9*H-*purin-6-amine hydrochloride;
and acceptable salt or prodrug thereof; as inhibitor of biofilm on a surface, particularly a surface of a biomaterial or of a medical device.

The most preferred inhibitor of biofilm on a surface is *N*-((1*R*,2*S*)-2-(3,4-difluorophenyl)cyclopropyl)-9-methyl-2-(propylthio)-9*H*-purin-6-amine as illustrated : 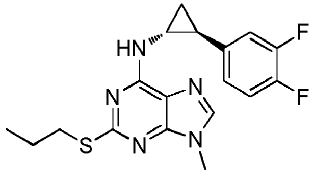 and acceptable salt or prodrug thereof

By surface one means any type of surface such as rubber or plastic surface as for example surface made of polyethylene, polypropylene, polyurethane, polyvinyl chloride, polyvinylpyrrolidone, polytetrafluoroethylene, silicone or the like, or copolymers but also and preferably metallic surface such as stainless steel, silver, gold, titanium, metallic alloys pyrolitic carbon, and the like. It can also be used on bioabsorbable or biomaterial surface such as biological prosthesis or devices which are made of biological material such as for example porcine or bovine pericardium

By inhibition of biofilm on a surface one means inhibition of the biofilm formation at all stages of its formation starting from a prevention or an inhibition of adherence of bacteria on the surface at step 1 but also and mainly an inhibition in bacteria grow, multiplication, and formation of microcolonies on the surface at step 2. By inhibition of biofilm one also means inhibition of the matrix at the maturation step 3 and inhibition of bacteria dispersion from the matrix in a colonisation step. By inhibition of biofilm, one also means killing bacteria at all steps of the biofilm formation.

By medical device one means biomaterial as defined above but also medical device requesting no bacterial contamination such as wound dressing, soft tissue fillers, root canal fillers, contact lens, blood bag and the like.

A last further aspect according to the invention, is a method for killing or controlling bacterial growth in biofilm formation on a surface comprising applying pyrimidine derivative on a surface either at a prevention step, reducing bacteria adherence and survival on the substrate or at a stage where the biofilm is already present, or even at a maturation step with a matrix formation wherein a more complex architecture of biofilm is established protecting bacteria as a barrier to conventional antibacterial agent.

The method of bacteria killing or prevention of bacterial growth on a surface is generally applied to biomaterials or medical devices.

The biomaterial or medical device are preferably implantable foreign material for clinical use in host mammals such as prosthetic devices, pacemakers, implantable cardioverter-defibrillators, intravascular catheters, coronary stent, heart valves, intraocular lens and the like but could be extended to other medical devices requesting no bacterial contamination such as for example wound dressings, soft tissue fillers containing local anaesthetics, root canal fillers with ancillary medicinal substances and the like.

The method of bacteria killing or prevention of bacterial growth could also be applied to surface of experimental device in need of such antibacterial treatment.

### Brief description of the Figures

**Figure 1** illustrates the inhibition of *Staphylococcus aureus* biofilm formation by the tested molecules (10 µM) at step 2 as compared to vehicle control (CTRL).
**Figure 2** illustrates a destruction of mature biofilm (step 3: 24-hour biofilm) by molecule 2329. Viable count of *S. epidermidis* biofilm after a 24h treatment with 2329.

### Detailed description of the invention

The invention is illustrated hereafter by the following non limiting examples.

### 1. Preparation of new pyrimidine derivatives :

### Example 1 : synthesis of N-((1R,2S)-2-(3,4-difluorophenyl)cyclopropyl)-9-methyl-2-(propylthio)-9H-purin-6-amine (1c)

### 6-Chloro-N⁴-methyl-2-(propylthio)pyrimidine-4,5-diamine (1a)

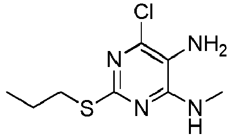

4,6-Dichloro-2-(propylthio)pyrimidin-5-amine (0.5 g, 2.1 mmol) was dissolved in methanol (2 mL) and supplemented with methylamine (192.0 mg, 6.3 mmol). The reaction mixture was introduced in a sealed vessel and heated at 100°C for 1 h. After concentration of the reaction mixture to dryness under vacuum, the residue was purified by silica gel column chromatography.
Yield: 96%.
Melting point: 119-121°C.
¹H NMR (DMSO-*d₆*) δ 0.95 (t, J=7.4 Hz, 3H, SCH₂CH₂C***H₃***), 1.64 (h, J=7.3 Hz, 2H, SCH₂C***H₂***CH₃), 2.87 (d, J=4.5 Hz, 3H, NHC***H₃***), 2.96 (t, J=7.2 Hz, 2H, SC***H₂***CH₂CH₃), 4.71 (s, 2H, *N**H₂***), 7.01 (q, J=4.4 Hz, 1H, N***H***CH₃). ¹³C NMR (DMSO-*d₆*) δ 13.3 (SCH₂CH₂***C***H₃), 22.6 (SCH₂***C***H₂CH₃), 27.8 (NH***C***H₃), 32.1 (S***C***H₂CH₂CH₃), 120.0 (C-5), 137.1 (C-6), 153.2 (C-4), 155.4 (C-2).

### 6-Chloro-9-methyl-2-(propylthio)-9H-purine (1b)

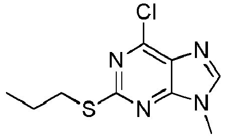

A solution of (**1a**) (233.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography.
Yield: 77%.
Melting point: 75-78°C.
¹H NMR (DMSO-*d₆*) δ 1.01 (t, J=7.4 Hz, 3H, SCH₂CH₂C***H₃***), 1.74 (h, J=7.3 Hz, 2H, SCH₂C***H₂***CH₃), 3.18 (t, J=7.2 Hz, 2H, SC***H₂***CH₂CH₃), 3.79 (s, 3H, NC***H₃***), 8.48 (s, 1H, 8-***H***).
¹³C NMR (DMSO-*d₆*) δ 13.2 (SCH₂CH₂***C***H₃), 22.0 (SCH₂***C***H₂CH₃), 29.9 (N***C***H₃), 32.6 (S***C***H₂CH₂CH₃), 127.9 (C-5), 147.0 (C-8), 148.7 (C-6), 153.2 (C-4), 163.9 (C-2).

### N-((1R,2S)-2-(3,4-Difluorophenyl)cyclopropyl)-9-methyl-2-(propylthio)-9H-purin-6-amine (1c)

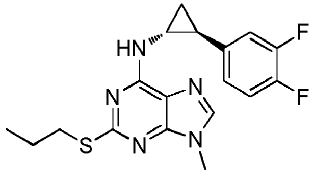

A solution of (**1b**) (122.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 42%.
Melting point: 94-96°C.
¹H NMR (CDCl₃) δ 0.93 (t, J=7.4 Hz, 3H, SCH₂CH₂C***H₃***), 1.32 (m, 2H, NHCH(C***H₂***)CHPh), 1.65 (h, J=7.3 Hz, 2H, SCH₂C***H₂***CH₃), 2.09 (m, 1H, NHCH(CH₂)C***H***Ph), 3.02 (m, 2H, SC***H₂***CH₂CH₃), 3.12 (bs, 1H, NHC***H***(CH₂)CHPh), 3.79 (s, 3H, NC***H₃***), 5.98 (bs, 1H, N***H***), 6.97 (m, 1H, 6'-H), 7.07 (m, 2H, 2'-H/5'-H), 7.59 (s, 1H, 8-***H***).
¹³C NMR (CDCl₃) δ 13.4 (SCH₂CH₂***C***H₃), 16.2 (NHCH(***C***H₂)CHPh), 22.8 (SCH₂***C***H₂CH₃), 25.2 (NHCH(CH₂)***C***HPh), 29.7 (N***C***H₃), 33.2 (S***C***H₂CH₂CH₃), 33.4 (NH***C***H(CH₂)CHPh), 115.5 (d, J=17 Hz, C-2'), 116.9 (d, J=17 Hz, C-5'), 117.4 (C-5), 122.6 (C-6'), 137.9 (C-1'), 139.5 (C-8), 147.9-149.9 (dd, 246 Hz/13 Hz, C-4'), 149.2-151.2 (dd, 247 Hz/13 Hz, C-3'), 150.8 (C-4), 154.5 (C-6), 165.6 (C-2).

### Example 2: synthesis of 9-methyl-N-((1R,2S)-2-phenylcyclopropyl)-2-(propylthio)-9H-purin-6-amine (2c)

### 9-Methyl-N-((1R,2S)-2-phenylcyclopropyl)-2-(propylthio)-9H-purin-6-amine (2c)

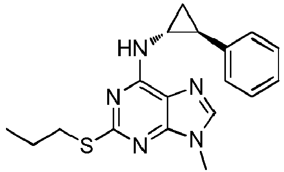

A solution of (**1b**) (122.0 mg, 0.5 mmol) in acetonitrile (2.5 mL) was supplemented with (1*R*,2*S*)-2-phenylcyclopropanamine (56.0 mg, 1.1 mmol) and triethylamine (0.13 mL) and then heated at 90°C under reflux for 4 h. After distillation of the solvents under vacuum, the residue was purified by silica gel column chromatography. Yield: 27%.
Melting point: 171-172.5°C.
¹H NMR (CDCl₃) δ 0.88 (t, J=7.4 Hz, 3H, SCH₂CH₂C***H₃***), 1.33 (m, 2H, NHCH(C***H₂***)CHPh), 1.60 (h, J=7.3 Hz,
2H, SCH₂C***H₂***CH₃), 2.13 (m, 1H, NHCH(CH₂)C***H***Ph), 2.92 (m, 1H, SC***H₂***CH₂CH₃), 3.06 (m, 1H, SC***H₂***CH₂CH₃), 3.22 (bs, 1H, NHC***H***(CH₂)CHPh), 3.75 (s, 3H, NC***H₃***), 5.97 (bs, 1H, N***H***), 7.19 (m, 3H, 2'-H/4'-H/6'-H), 7.30 (m, 2H, 3'-H/5'-H), 7.58 (s, 1H, 8-***H***).
¹³C NMR (CDCl₃) δ 13.3 (SCH₂CH₂***C***H₃), 16.8 (NHCH(***C***H₂)CHPh), 22.8 (SCH₂***C***H₂CH₃), 25.7 (NHCH(CH₂)***C***HPh), 29.7 (N***C***H₃), 33.3 (S***C***H₂CH₂CH₃), 33.5 (NH***C***H(CH₂)CHPh), 117.4 (C-5), 126.0 (C-4'), 126.2 (C-2'/C-6'), 128.3 (C-3'/C-5'), 139.5 (C-8), 140.9 (C-1'), 150.8 (C-4), 154.6 (C-6), 165.6 (C-2).

### Example 3 : synthesis of N-((1R,2S)-2-(3,4-difluorophenyl)cyclopropyl)-9-ethyl-2-(propylthio)-9H-purin-6-amine (3c)

### 6-Chloro-N⁴-ethyl-2-(propylthio)pyrimidine-4,5-diamine (3a)

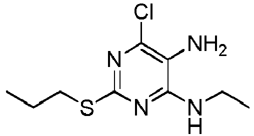

4,6-Dichloro-2-(propylthio)pyrimidin-5-amine (0.5 g, 2.1 mmol) was dissolved in methanol (2 mL) and supplemented with ethylamine (284.0 mg, 6.3 mmol). The reaction mixture was introduced in a sealed vessel and heated at 100°C for 1 h. After concentration of the reaction mixture to dryness under vacuum, the residue was purified by silica gel column chromatography.
Yield: 77%.
Melting point: 96-98°C.
¹H NMR (DMSO-*d₆*) δ 0.95 (t, J=7.4 Hz, 3H, SCH₂CH₂C***H₃***), 1.16 (t, J=7.2 Hz, 3H, NHCH₂C***H₃***), 1.63 (h, J=7.3 Hz, 2H, SCH₂C***H₂***CH₃), 2.94 (t, J=7.2 Hz, 2H, SC***H₂***CH₂CH₃), 3.37 (m, 2H, NHC***H₂***CH₃), 4.75 (s, 2H, *N**H₂***), 6.95 (t, J=4.8 Hz, 1H, N***H***CH₂CH₃).
¹³C NMR (DMSO-*d₆*) δ 13.3 (SCH₂CH₂***C***H₃), 14.3 (NHCH₂***C***H₃), 22.7 (SCH₂***C***H₂CH₃), 32.1 (S***C***H₂CH₂CH₃), 35.7 (NH***C***H₂CH₃), 119.8 (C-5), 137.3 (C-6), 152.5 (C-4), 155.3 (C-2).

### 6-Chloro-9-ethyl-2-(propylthio)-9H-purine (3b)

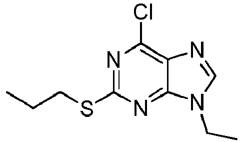

A solution of (**3a**) (247.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography.
Yield: 77%.
Melting point: 96-97.5°C.
¹H NMR (DMSO-*d₆*) δ 1.01 (t, J=7.4 Hz, 3H, SCH₂CH₂C***H₃***), 1.44 (t, J=7.3 Hz, 3H, NCH₂C***H₃***), 1.74 (h, J=7.3 Hz, 2H, SCH₂C***H₂***CH₃), 3.17 (t, J=7.2 Hz, 2H, SC***H₂***CH₂CH₃), 4.24 (q, J=7.3 Hz, 2H, NC***H₂***CH₃), 8.56 (s, 1H, 8-***H).***
¹³C NMR (DMSO-*d₆*) δ 13.2 (SCH₂CH₂***C***H₃), 14.6 (NCH₂***C***H₃), 22.0 (SCH₂***C***H₂CH₃), 32.6 (S***C***H₂CH₂CH₃), 39.9 (N***C***H₂CH₃), 128.1 (C-5), 146.0 (C-8), 148.8 (C-6), 152.7 (C-4), 163.8 (C-2).

### N-((1R,2S)-2-(3,4-Difluorophenyl)cyclopropyl)-9-ethyl-2-(propylthio)-9H-purin-6-amine (3c)

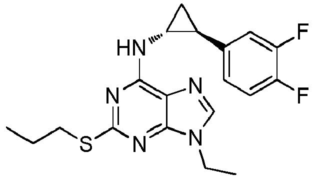

A solution of (**3b**) (129.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 43%.
Melting point: 109.5-111.5°C.
¹H NMR (CDCl₃) δ 0.94 (t, J=7.4 Hz, 3H, SCH₂CH₂C***H₃***), 1.32 (m, 2H, NHCH(C***H₂***)CHPh), 1.50 (t, j=7.3 Hz, 3H, NCH₂C***H₃***), 1.67 (h, J=7.3 Hz, 2H, SCH₂C***H₂***CH₃), 2.09 (m, 1H, NHCH(CH₂)C***H***Ph), 3.03 (m, 2H, SC***H₂***CH₂CH₃), 3.12 (bs, 1H, NHC***H***(CH₂)CHPh), 4.18 (q, J=7.3 Hz, 2H, NC***H₂***CH₃), 5.90 (bs, 1H, NH), 6.99 (m, 1H, 6'-H), 7.08 (m, 2H, 2'-H/5'-H), 7.63 (s, 1H, 8-***H***).
¹³C NMR (CDCl₃) δ 13.4 (SCH₂CH₂***C***H₃), 15.5 (NCH₂***C***H₃), 16.1 (NHCH(***C***H₂)CHPh), 22.9 (SCH₂***C***H₂CH₃), 25.2 (NHCH(CH₂)***C***HPh), 33.2 (S***C***H₂CH₂CH₃), 33.3 (NH***C***H(CH₂)CHPh), 38.7 (N***C***H₂CH₃), 115.6 (d, J=17 Hz, C-2'), 116.9 (d, J=17 Hz, C-5'), 117.6 (C-5), 122.7 (C-6'), 137.9 (C-1'), 138.5 (C-8), 147.9-149.9 (dd, 246 Hz/13 Hz, C-4'), 149.2-151.2 (dd, 247 Hz/13 Hz, C-3'), 150.3 (C-4), 154.5 (C-6), 165.4 (C-2).

### Example 4: synthesis of N-((1R,2S)-2-(3,4-difluorophenyl)cyclopropyl)-9-propyl-2-(propylthio)-9H-purin-6-amine (4c)

### 6-Chloro-N⁴-propyl-2-(propylthio)pyrimidine-4,5-diamine (4a)

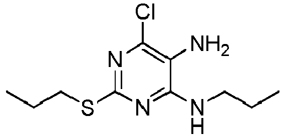

4,6-Dichloro-2-(propylthio)pyrimidin-5-amine (0.5 g, 2.1 mmol) was dissolved in methanol (2 mL) and supplemented with *n*-propylamine (370.0 mg, 6.3 mmol). The reaction mixture was introduced in a sealed vessel and heated at 100°C for 30 min. After concentration of the reaction mixture to dryness under vacuum, the residue was purified by silica gel column chromatography.
Yield: 91%.
Melting point: 100-102°C.
¹H NMR (DMSO-*d₆*) δ 0.91 (t, J=7.4 Hz, 3H, NHCH₂CH₂C***H₃***), 0.95 (t, J=7.3 Hz, 3H, SCH₂CH₂C***H₃***), 1.56 (h, J=7.3 Hz, 2H, NHCH₂C***H₂***CH₃), 1.64 (h, J=7.3 Hz, 2H, SCH₂C***H₂***CH₃), 2.93 (t, J=7.2 Hz, 2H, SC***H₂***CH₂CH₃), 3.32 (m, 2H, NHC***H₂***CH₂CH₃), 4.76 (s, 2H, *N**H₂***), 6.96 (t, J=4.8 Hz, 1H, N***H***CH₂CH₂CH₃).
¹³C NMR (DMSO-*d₆*) δ 11.5 (NHCH₂CH₂***C***H₃), 13.3 (SCH₂CH₂***C***H₃), 21.9 (NHCH₂***C***H₂CH₃), 22.8 (SCH₂***C***H₂CH₃), 32.1 (S***C***H₂CH₂CH₃), 42.7 (NH***C***H₂CH₂CH₃), 119.8 (C-5), 137.3 (C-6), 152.6 (C-4), 155.2 (C-2).

### 6-Chloro-9-propyl-2-(propylthio)-9H-purine (4b)

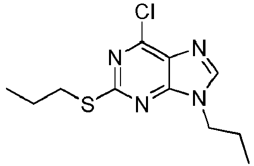

A solution of (**4a**) (261.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography.
Yield: 94%.
Melting point: liquid.
¹H NMR (DMSO-*d₆*) δ 0.85 (t, J=7.4 Hz, 3H, NCH₂CH₂C***H₃***), 1.01 (t, J=7.4 Hz, 3H, SCH₂CH₂C***H₃***), 1.74 (h, J=7.3 Hz, 2H, SCH₂C***H₂***CH₃), 1.86 (h, J=7.3 Hz, 2H, NCH₂C***H₂***CH₃), 3.17 (t, J=7.2 Hz, 2H, SC***H₂***CH₂CH₃), 4.18 (t, J=7.0 Hz, 2H, NC***H₂***CH₂CH₃), 8.55 (s, 1H, 8-***H***).
¹³C NMR (DMSO-*d₆*) δ 10.9 (NCH₂CH₂***C***H₃), 13.2 (SCH₂CH₂***C***H₃), 22.1 (SCH₂***C***H₂CH₃), 22.3 (NCH₂***C***H₂CH₃), 32.6 (S***C***H₂CH₂CH₃), 45.3 (NCH₂CH₂***C***H₃), 128.0 (C-5), 146.4 (C-8), 148.9 (C-6), 152.9 (C-4), 163.8 (C-2).

### N-((1R,2S)-2-(3,4-Difluorophenyl)cyclopropyl)-9-propyl-2-(propylthio)-9H-purin-6-amine (4c)

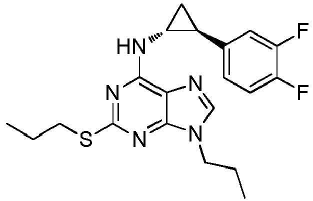

A solution of (**4b**) (136.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 45%.
Melting point: 97-99°C.
¹H NMR (CDCl₃) δ 0.94 (t, J=7.4 Hz, 6H, NCH₂CH₂C***H₃***/SCH₂CH₂C***H₃***), 1.32 (m, 2H, NHCH(C***H₂***)CHPh), 1.67 (h, J=7.3 Hz, 2H, SCH₂C***H₂***CH₃), 1.90 (h, J=7.4 Hz, 2H, NCH₂C***H₂***CH₃), 2.09 (m, 1H, NHCH(CH₂)C***H***Ph), 3.03 (m, 2H, SC***H₂***CH₂CH₃), 3.11 (bs, 1H, NHC***H***(CH₂)CHPh), 4.09 (t, J=7.1 Hz, 2H, NC***H₂***CH₂CH₃), 5.86 (bs, 1H, N***H***), 7.00 (m, 1H, 6'-H), 7.09 (m, 2H, 2'-H/5'-H), 7.61 (s, 1H, 8-***H***).
¹³C NMR (CDCl₃) δ 11.2 (NCH₂CH₂***C***H₃), 13.4 (SCH₂CH₂***C***H₃), 15.7 (NHCH(***C***H₂)CHPh), 22.9 (SCH₂***C***H₂CH₃), 23.3 (NCH₂CH₂***C***H₃), 25.2 (NHCH(CH₂)***C***HPh), 33.2 (S***C***H₂CH₂CH₃), 33.3 (NH***C***H(CH₂)CHPh), 45.3 (NCH₂CH₂***C***H₃), 115.6 (d, J=17 Hz, C-2'), 116.9 (d, J=17 Hz, C-5'), 117.6 (C-5), 122.7 (C-6'), 137.9 (C-1'), 139.0 (C-8),147.9-149.9 (dd, 246 Hz/13 Hz, C-4'), 149.2-151.2 (dd, 247 Hz/13 Hz, C-3'), 150.5 (C-4), 154.5 (C-6), 165.4 (C-2).

### Example 5 : synthesis of N-((1R,2S)-2-(3,4-difluorophenyl)cyclopropyl)-9-isopropyl-2-(propylthio)-9H-purin-6-amine (5c)

### 6-Chloro-N⁴-isopropyl-2-(propylthio)pyrimidine-4,5-diamine (5a)

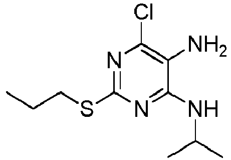

4,6-Dichloro-2-(propylthio)pyrimidin-5-amine (0.5 g, 2.1 mmol) was dissolved in methanol (2 mL) and supplemented with isopropylamine (370.0 mg, 6.3 mmol). The reaction mixture was introduced in a sealed vessel and heated at 100°C for 90 min. After concentration of the reaction mixture to dryness under vacuum, the residue was purified by silica gel column chromatography.
Yield: 95%.
Melting point: 81-83°C.
¹H NMR (DMSO-*d₆*) δ 0.95 (t, J=7.3 Hz, 3H, SCH₂CH₂C***H₃***), 1.19 (d, J=6.5 Hz, 6H, NHCH(C***H₃***)*₂*), 1.63 (h, J=7.3 Hz, 2H, SCH₂C***H₂***CH₃), 2.93 (t, J=7.2 Hz, 2H, SC***H₂***CH₂CH₃), 4.16 (m, 1H, NHC***H***(CH₃)₂), 4.81 (s, 2H, *N**H₂***), 6.69 (d, J=4.8 Hz, 1H, N***H***CH(CH₃)₂).
¹³C NMR (DMSO-*d₆*) δ 13.3 (SCH₂CH₂***C***H₃), 22.2 (NHCH(***C***H₃)₂), 22.8 (SCH₂***C***H₂CH₃), 32.1 (S***C***H₂CH₂CH₃), 42.6 (NH***C***H(CH₃)₂), 119.7 (C-5), 137.3 (C-6), 151.7 (C-4), 155.1 (C-2).

### 6-Chloro-9-isopropyl-2-(propylthio)-9H-purine (5b)

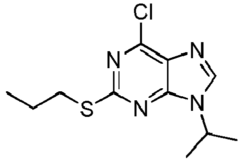

A solution of (**5a**) (261.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography. Yield: 37%.
Melting point: 121-122.5°C.
¹H NMR (DMSO-*d₆*) δ 1.01 (t, J=7.4 Hz, 3H, SCH₂CH₂C***H₃***), 1.56 (d, J=6.8 Hz, 6H, NCH(C***H₃***)*₂*), 1.74 (h, J=7.3 Hz, 2H, SCH₂C***H₂***CH₃), 3.16 (t, J=7.2 Hz, 2H, SC***H₂***CH₂CH₃), 4.80 (hept, J=6.8 Hz, 1H, NC***H***(CH₃)₂), 8.62 (s, 1H, 8-***H***).
¹³C NMR (DMSO-*d₆*) δ 13.3 (SCH₂CH₂***C***H₃), 21.7 (NCH(***C***H₃)₂), 22.1 (SCH₂***C***H₂CH₃), 32.6 (S***C***H₂CH₂CH₃), 47.9 (N***C***H(CH₃)₂), 128.4 (C-5), 144.7 (C-8), 148.9 (C-6), 152.3 (C-4), 163.5 (C-2).
*N*-((1*R*,2*S*)-2-(3,4-Difluorophenyl)cyclopropyl)-9-isopropyl-2-(propylthio)-9*H*-purin-6-amine (**5c**)

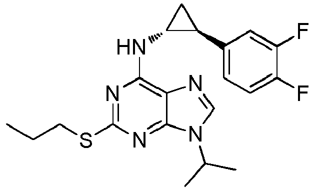

A solution of (**5b**) (136.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 47%.
Melting point: 98.5-100.5°C.
¹H NMR (CDCl₃) δ 0.95 (t, J=7.4 Hz, 3H, SCH₂CH₂C***H₃***), 1.31 (m, 2H, NHCH(C***H₂***)CHPh), 1.58 (dd, j=6.8 Hz/1.6 Hz, 6H, NCH(C***H₃***)₂), 1.68 (h, J=7.3 Hz, 2H, SCH₂C***H₂***CH₃), 2.08 (m, 1H, NHCH(CH₂)C***H***Ph), 3.04 (m, 2H, SC***H₂***CH₂CH₃), 3.10 (bs, 1H, NHC***H***(CH₂)CHPh), 4.77 (hept, J=6.8 Hz, 1H, NC***H***(CH₃)₂), 5.95 (bs, 1H, N***H***), 7.00 (m, 1H, 6'-H), 7.09 (m, 2H, 2'-H/5'-H), 7.68 (s, 1H, 8-***H***).
¹³C NMR (CDCl₃) δ 13.5 (SCH₂CH₂***C***H₃), 16.0 (NHCH(***C***H₂)CHPh), 22.6 (NCH(***C***H₃)₂), 22.9 (SCH₂***C***H₂CH₃), 25.2 (NHCH(CH₂)***C***HPh), 33.2 (S***C***H₂CH₂CH₃), 33.3 (NH***C***H(CH₂)CHPh), 47.0 (N***C***H(CH₃)₂), 115.7 (d, J=17 Hz, C-2'), 116.9 (d, J=17 Hz, C-5'), 117.9 (C-5), 122.8 (C-6'), 136.7 (C-8), 137.9 (C-1'), 147.9-149.9 (dd, 246 Hz/13 Hz, C-4'), 149.2-151.2 (dd, 247 Hz/13 Hz, C-3'), 150.0 (C-4), 154.6 (C-6), 165.1 (C-2).

### Example 6: synthesis of 9-cyclopropyl-N-((1R,2S)-2-(3,4-difluorophenyl)cyclopropyl)-2-(propylthio)-9H-purin-6-amine (6c)

### 6-Chloro-N⁴-cyclopropyl-2-(propylthio)pyrimidine-4,5-diamine (6a)

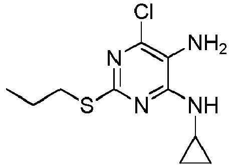

4,6-Dichloro-2-(propylthio)pyrimidin-5-amine (0.5 g, 2.1 mmol) was dissolved in methanol (2 mL) and supplemented with cyclopropylamine (360.0 mg, 6.3 mmol). The reaction mixture was introduced in a sealed vessel and heated at 100°C for 30 min. After concentration of the reaction mixture to dryness under vacuum, the residue was purified by silica gel column chromatography.
Yield: 92%.
Melting point: 96-98°C.
¹H NMR (DMSO-*d₆*) δ 0.49 (s, 2H, NHCH(C***H₂***)*₂*), 0.73 (d, J=5.7 Hz, 2H, NHCH(C***H₂***)*₂*), 0.95 (t, J=7.1 Hz, 3H, SCH₂CH₂C***H₃***), 1.66 (h, J=7.0 Hz, 2H, SCH₂C***H₂***CH₃), 2.80 (m, 1H, NHC***H***(CH₂)₂), 2.97 (t, J=6.9 Hz, 2H, SC***H₂***CH₂CH₃), 4.74 (s, 2H, *N**H₂***), 7.09 (s, 1H, N***H***CH(CH₂)₂).
¹³C NMR (DMSO-*d₆*) δ 6.2 (NHCH(***C***H₂)₂), 13.3 (SCH₂CH₂***C***H₃), 22.8 (SCH₂***C***H₂CH₃), 24.1 (NH***C***H(CH₂)₂), 32.2 (S***C***H₂CH₂CH₃), 120.0 (C-5), 137.3 (C-6), 153.4 (C-4), 155.2 (C-2).

### 6-Chloro-9-cyclopropyl-2-(propylthio)-9H-purine (6b)

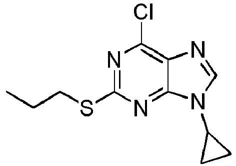

A solution of (6a) (259.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography.
Yield: 58%.
Melting point: 128.5-130°C.
¹H NMR (DMSO-*d₆*) δ 1.02 (t, J=7.4 Hz, 3H, SCH₂CH₂C***H₃***), 1.09 (m, 2H, NCH(C***H₂***)*₂*), 1.16 (m, 2H, NCH(C***H₂***)*₂*), 1.75 (h, J=7.3 Hz, 2H, SCH₂C***H₂***CH₃), 3.17 (t, J=7.2 Hz, 2H, SC***H₂***CH₂CH₃), 3.55 (m, 1H, NC***H***(CH₂)₂), 8.52 (s, 1H, 8-***H***).
¹³C NMR (DMSO-*d₆*) δ 5.4 (NCH(***C***H₂)₂), 13.3 (SCH₂CH₂***C***H₃), 22.1 (SCH₂***C***H₂CH₃), 25.6 (N***C***H(CH₂)₂), 32.7 (S***C***H₂CH₂CH₃), 128.2 (C-5), 146.7 (C-8), 148.8 (C-6), 153.9 (C-4), 163.9 (C-2).

### 9-Cyclopropyl-N-((1R,2S)-2-(3,4-difluorophenyl)cyclopropyl)-2-(propylthio)-9H-purin-6-amine (6c)

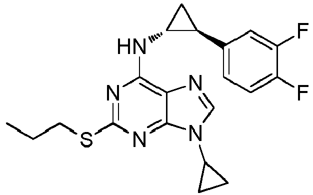

A solution of (**6b**) (135.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 40%.
Melting point: 137-139°C.
¹H NMR (CDCl₃) δ 0.95 (t, J=7.4 Hz, 3H, SCH₂CH₂C***H₃***), 1.10 (m, 2H, NCH(C***H₂***)₂), 1.13 (m, 2H, NCH(C***H₂***)₂), 1.31 (m, 2H, NHCH(C***H₂***)CHPh), 1.68 (h, J=7.3 Hz, 2H, SCH₂C***H₂***CH₃), 2.08 (m, 1H, NHCH(CH₂)C***H***Ph), 3.03 (m, 2H, SC***H₂***CH₂CH₃), 3.10 (bs, 1H, NHC***H***(CH₂)CHPh), 3.38 (tt, J=7.1 Hz/3.9 Hz, 1H, NC***H***(CH₂)₂), 5.88 (bs, 1H, N***H***), 6.99 (m, 1H, 6'-H), 7.08 (m, 2H, 2'-H/5'-H), 7.61 (s, 1H, 8-***H***).
¹³C NMR (CDCl₃) δ 5.93 (NCH(***C***H₂)₂), 13.5 (SCH₂CH₂***C***H₃), 16.1 (NHCH(***C***H₂)CHPh), 22.9 (SCH₂***C***H₂CH₃), 25.2 (NHCH(CH₂)***C***HPh), 25.3 (N***C***H(CH₂)₂), 33.2 (S***C***H₂CH₂CH₃), 33.3 (NH***C***H(CH₂)CHPh), 115.6 (d, J=17 Hz, C-2'), 116.9 (d, J=17 Hz, C-5'), 117.4 (C-5), 122.7 (C-6'), 137.9 (C-1'), 139.4 (C-8),147.9-149.9 (dd, 246 Hz/13 Hz, C-4'), 149.2-151.2 (dd, 247 Hz/13 Hz, C-3'), 151.6 (C-4), 154.4 (C-6), 165.6 (C-2).

### Example 7 : synthesis of 9-butyl-N-((1R,2S)-2-(3,4-difluorophenyl)cyclopropyl)-2-(propylthio)-9H-purin-6-amine (7c)

### N⁴-Butyl-6-chloro-2-(propylthio)pyrimidine-4,5-diamine (7a)

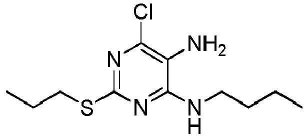

4,6-Dichloro-2-(propylthio)pyrimidin-5-amine (0.5 g, 2.1 mmol) was dissolved in methanol (2 mL) and supplemented with *n*-butylamine (460.0 mg, 6.3 mmol). The reaction mixture was introduced in a sealed vessel and heated at 100°C for 30 min. After concentration of the reaction mixture to dryness under vacuum, the residue was purified by silica gel column chromatography.
Yield: 95%.
Melting point: liquid.
¹H NMR (DMSO-*d₆*) δ 0.91 (t, J=7.4 Hz, 3H, NHCH₂CH₂CH₂C***H₃***), 0.95 (t, J=7.4 Hz, 3H, SCH₂CH₂C***H₃***), 1.35 (h, J=7.4 Hz, 2H, NHCH₂CH₂C***H₂***CH₃), 1.55 (p, J=7.5 Hz, 2H, NHCH₂C***H₂***CH₂CH₃), 1.64 (h, J=7.4 Hz, 2H, SCH₂C***H₂***CH₃), 2.95 (t, J=7.3 Hz, 2H, SC***H₂***CH₂CH₃), 3.37 (m, 2H, NHC***H₂***CH₂CH₂CH₃), 4.76 (s, 2H, *N**H₂***), 6.94 (t, J=5.2 Hz, 1H, N***H***CH₂CH₂CH₂CH₃).
¹³C NMR (DMSO-*d₆*) δ 13.3 (SCH₂CH₂***C***H₃), 13.7 (NHCH₂CH₂CH₂***C***H₃), 19.6 (NHCH₂CH₂***C***H₂CH₃), 22.8 (SCH₂***C***H₂CH₃), 30.8 (NHCH₂***C***H₂CH₂CH₃), 32.1 (S***C***H₂CH₂CH₃), 40.6 (NH***C***H₂CH₂CH₂CH₃), 119.8 (C-5), 137.3 (C-6), 152.6 (C-4), 155.2 (C-2).

### 9-Butyl-6-chloro-2-(propylthio)-9H-purine (7b)

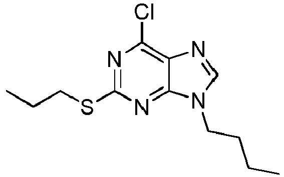

A solution of (**7a**) (275.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography. Yield: 72%.
Melting point: liquid.
¹H NMR (DMSO-*d*₆) δ 0.90 (t, J=7.4 Hz, 3H, NCH₂CH₂CH₂C***H₃***), 1.01 (t, J=7.4 Hz, 3H, SCH₂CH₂C***H₃***), 1.26 (h, J=7.4 Hz, 2H, NCH₂CH₂C***H₂***CH₃), 1.74 (h, J=7.3 Hz, 2H, SCH₂C***H₂***CH₃), 1.83 (p, J=7.2 Hz, 2H, NCH₂C***H₂***CH₂CH₃), 3.16 (t, J=7.2 Hz, 2H, SC***H₂***CH₂CH₃), 4.22 (t, J=7.1 Hz, 2H, NC***H₂***CH₂CH₂CH₃), 8.56 (s, 1H, 8-***H***).
¹³C NMR (DMSO-*d₆*) δ 13.2 (SCH₂CH₂***C***H₃), 13.3 (NCH₂CH₂CH₂***C***H₃), 19.2 (NCH₂CH₂***C***H₂CH₃), 22.1 (SCH₂***C***H₂CH₃), 30.9 (NCH₂***C***H₂CH₂CH₃), 32.6 (S***C***H₂CH₂CH₃), 43.3 (N***C***H₂CH₂CH₂CH₃), 127.9 (C-5), 146.3 (C-8), 148.9 (C-6), 152.8 (C-4), 163.8 (C-2).

### 9-Butyl-N-((1R,2S)-2-(3,4-difluorophenyl)cyclopropyl)-2-(propylthio)-9H-purin-6-amine (7c)

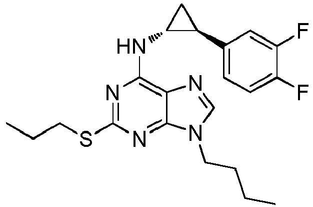

A solution of (**7b**) (143.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 61%.
Melting point: 85-87°C.
¹H NMR (CDCl₃) δ 0.95 (m, 6H, NCH₂CH₂CH₂C***H₃***/SCH₂CH₂C***H₃***), 1.33 (m, 4H,
NCH₂CH₂C***H₂***CH₃/NHCH(C***H₂***)CHPh), 1.68 (h, J=7.3 Hz, 2H, SCH₂C***H₂***CH₃), 1.86 (p, J=7.3 Hz, 2H, NCH₂C***H₂***CH₂CH₃), 2.10 (m, 1H, NHCH(CH₂)C***H***Ph), 3.03 (m, 2H, SC***H₂***CH₂CH₃), 3.13 (bs, 1H, NHC***H***(CH₂)CHPh), 4.14 (t, J=7.1 Hz, 2H, NC***H***₂CH₂CH₂CH₃), 6.19 (bs, 1H, N***H***), 6.99 (m, 1H, 6'-H), 7.08 (m, 2H, 2'-H/5'-H), 7.64 (s, 1H, 8-***H***).
¹³C NMR (CDCl₃) δ 13.5 (SCH₂CH₂***C***H₃/NCH₂CH₂CH₂***C***H₃), 16.0 (NHCH(***C***H₂)CHPh), 19.8 (NCH₂CH₂***C***H₂CH₃), 22.9 (SCH₂***C***H₂CH₃), 25.2 (NHCH(CH₂)***C***HPh), 31.9 (NCH₂***C***H₂CH₂CH₃), 33.2 (S***C***H₂CH₂CH₃), 33.4 (NH***C***H(CH₂)CHPh), 43.5 (N***C***H₂CH₂CH₂CH₃), 115.7 (d, J=17 Hz, C-2'), 116.9 (d, J=17 Hz, C-5'), 117.5 (C-5), 122.7 (C-6'), 136.5 (C-8), 137.9 (C-1'), 147.9-149.9 (dd, 246 Hz/13 Hz, C-4'), 149.2-151.2 (dd, 247 Hz/13 Hz, C-3'), 150.4 (C-4), 154.3 (C-6), 165.8 (C-2).

### Example 8 : synthesis of 9-(sec-butyl)-N-((1R,2S)-2-(3,4-difluorophenyl)cyclopropyl)-2-(propylthio)-9H-purin-6-amine (8c)

### N⁴-(sec-Butyl)-6-chloro-2-(propylthio)pyrimidine-4,5-diamine (8a)

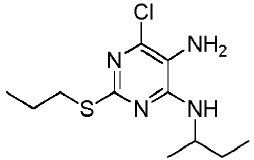

4,6-Dichloro-2-(propylthio)pyrimidin-5-amine (0.5 g, 2.1 mmol) was dissolved in methanol (2 mL) and supplemented with *sec*-butylamine (460.0 mg, 6.3 mmol). The reaction mixture was introduced in a sealed vessel and heated at 100°C for 90 min. After concentration of the reaction mixture to dryness under vacuum, the residue was purified by silica gel column chromatography.
Yield: 81%.
Melting point: liquid.
¹H NMR (DMSO-*d₆*) δ 0.87 (t, J=7.4 Hz, 3H, NHCH(CH₃)CH₂C***H₃***), 0.95 (t, J=7.3 Hz, 3H, SCH₂CH₂C***H₃***), 1.15 (d, J=6.6 Hz, 3H, NHCH(C***H₃***)CH₂CH₃), 1.53 (m, 2H, NHCH(CH₃)C***H₂***CH₃), 1.64 (h, J=7.3 Hz, 2H, SCH₂C***H₂***CH₃), 2.93 (t, J=7.2 Hz, 2H, SC***H₂***CH₂CH₃), 4.01 (hept, J=6.6 Hz, 1H, NHC***H***(CH₃)CH₂CH₃), 4.81 (s, 2H, *N**H₂***), 6.64 (d, J=7.5 Hz, 1H, N***H***CH(CH₃)CH₂CH₃).
¹³C NMR (DMSO-*d₆*) δ 10.5 (NHCH(CH₃)CH₂***C***H₃), 13.3 (SCH₂CH₂***C***H₃), 19.8 (NHCH(***C***H₃)CH₂CH₃), 22.8 (SCH₂***C***H₂CH₃), 28.6 (NHCH(CH₃)***C***H₂CH₃), 32.1 (S***C***H₂CH₂CH₃), 47.9 (NH***C***H(CH₃)CH₂CH₃), 119.7 (C-5), 137.3 (C-6), 152.0 (C-4), 155.0 (C-2).

### 9-(sec-Butyl)-6-chloro-2-(propylthio)-9H-purine (8b)

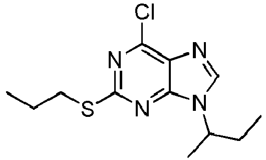

A solution of (**8a**) (275.0 mg, 1 mmol) in acetic acid (2.5 mL) and triethyl orthoformate (2.5 mL, 15 mmol) was heated at a temperature of 130°C under reflux for 4 h. After distillation of the acid acetic and triethyl orthoformate under vacuum, the residue was purified by silica gel column chromatography. Yield: 39%.
Melting point: 64-66°C.
¹H NMR (DMSO-*d₆*) δ 0.74 (t, J=7.4 Hz, 3H, NCH(CH₃)CH₂C***H₃***), 1.01 (t, J=7.4 Hz, 3H, SCH₂CH₂C***H₃***), 1.57 (d, J=6.9 Hz, 3H, NCH(C***H₃***)CH₂CH₃), 1.74 (h, J=7.3 Hz, 2H, SCH₂C***H₂***CH₃), 1.95 (m, 2H, NCH(CH₃)C***H₂***CH₃), 3.15 (m, 2H, SC***H₂***CH₂CH₃), 4.57 (m, 1H, NC***H***(CH₃)CH₂CH₃), 8.63 (s, 1H, 8-***H***).
¹³C NMR (DMSO-*d₆*) δ 10.5 (NCH(CH₃)CH₂***C***H₃), 13.3 (SCH₂CH₂***C***H₃), 19.8 (NCH(***C***H₃)CH₂CH₃), 22.1 (SCH₂***C***H₂CH₃), 28.3 (NCH(CH₃)***C***H₂CH₃), 32.6 (S***C***H₂CH₂CH₃), 53.6 (N***C***H(CH₃)CH₂CH₃), 128.2 (C-5), 145.1 (C-8), 149.0 (C-6), 152.5 (C-4), 163.6 (C-2).

### 9-(sec-Butyl)-N-((1R,2S)-2-(3,4-difluorophenyl)cyclopropyl)-2-(propylthio)-9H-purin-6-amine (8c)

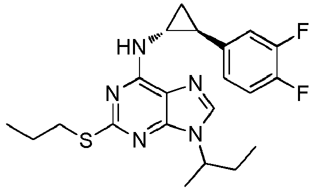

A solution of (**8b**) (143.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 4 h. After distillation of the acetonitrile and triethylamine under vacuum, the residue was purified by silica gel column chromatography.
Yield: 66%.
Melting point: 68-71°C.
¹H NMR (CDCl₃) δ 0.85 (td, J=7.4 Hz/1.6 Hz, 3H, NCH(CH₃)CH₂C***H₃***), 0.96 (t, J=7.3 Hz, 3H, SCH₂CH₂C***H₃***), 1.33 (m, 2H, NHCH(C***H₂***)CHPh), 1.57 (d, J=6.9 Hz, 3H, NCH(C***H₃***)CH₂CH₃), 1.69 (h, J=7.3 Hz, 2H, SCH₂C***H₂***CH₃), 1.95 (m, 2H, NCH(CH₃)C***H₂***CH₃), 2.10 (m, 1H, NHCH(CH₂)C***H***Ph), 3.03 (m, 2H, SC***H₂***CH₂CH₃), 3.11 (bs, 1H, NHC***H***(CH₂)CHPh), 4.52 (m, 1H, NC***H***(CH₃)CH₂CH₃), 6.12 (bs, 1H, N***H***), 7.00 (m, 1H, 6'-H), 7.10 (m, 2H, 2'-H/5'-H), 7.67 (s, 1H, 8-***H***).
¹³C NMR (CDCl₃) δ 10.7 (NCH(CH₃)CH₂***C***H₃), 13.5 (SCH₂CH₂***C***H₃), 15.9 (NHCH(***C***H₂)CHPh), 20.6 (NCH(***C***H₃)CH₂CH₃), 22.9 (SCH₂***C***H₂CH₃), 25.2 (NHCH(CH₂)***C***HPh), 29.5 (NCH(CH₃)***C***H₂CH₃), 33.2 (S***C***H₂CH₂CH₃), 33.3 (NH***C***H(CH₂)CHPh), 53.0 (N***C***H(CH₃)CH₂CH₃), 115.8 (d, J=17 Hz, C-2'), 116.9 (d, J=17 Hz, C-5'), 117.3 (C-5), 122.8 (C-6'), 137.0 (C-8), 137.9 (C-1'), 147.9-149.9 (dd, 246 Hz/13 Hz, C-4'), 149.2-151.2 (dd, 247 Hz/13 Hz, C-3'), 150.3 (C-4), 154.4 (C-6), 165.1 (C-2).

### Example 9 : synthesis of 9-(tert-butyl)-N-((1R,2S)-2-(3,4-difluorophenyl)cyclopropyl)-2-(propylthio)-9H-purin-6-amine (9c)

### N⁴-(tert-Butyl)-6-chloro-2-(propylthio)pyrimidine-4,5-diamine (9a)

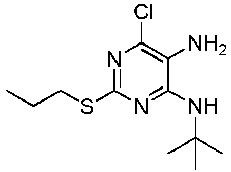

4,6-Dichloro-2-(propylthio)pyrimidin-5-amine (0.5 g, 2.1 mmol) was dissolved in methanol (2 mL) and supplemented with *tert*-butylamine (460.0 mg, 6.3 mmol). The reaction mixture was introduced in a sealed vessel and heated at 100°C for 24 h. After concentration of the reaction mixture to dryness under vacuum, the residue was purified by silica gel column chromatography.
Yield: 88%.
Melting point: 88-89°C.
¹H NMR (DMSO-*d₆*) δ 0.95 (t, J=7.3 Hz, 3H, SCH₂CH₂C***H₃***), 1.43 (s, 9H, NHC(C***H₃***)*₃*), 1.62 (h, J=7.3 Hz, 2H, SCH₂C***H₂***CH₃), 2.95 (t, J=7.3 Hz, 2H, SC***H₂***CH₂CH₃), 4.91 (bs, 2H, *N**H₂***), 6.19 (s, 1H, N***H***C(CH₃)₃).
¹³C NMR (DMSO-*d₆*) δ 13.3 (SCH₂CH₂***C***H₃), 22.9 (SCH₂***C***H₂CH₃), 28.5 (NHC(***C***H₃)₃), 31.9 (S***C***H₂CH₂CH₃), 51.9 (NH***C***(CH₃)₃), 120.3 (C-5), 137.6 (C-6), 152.1 (C-4), 154.5 (C-2).

### 9-(tert-Butyl)-6-chloro-2-(propylthio)-9H-purine (9b)

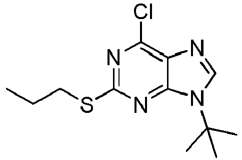

A solution of (**9a**) (275.0 mg, 1 mmol) in acetic acid (2.5 mL) and triethyl orthoformate (2.5 mL, 15 mmol) was heated at a temperature of 130°C under reflux for 10 h. After distillation of the acid acetic and triethyl orthoformate under vacuum, the residue was purified by silica gel column chromatography. Yield: 41%.
Melting point: 116-117°C.
¹H NMR (DMSO-*d₆*) δ 1.02 (t, J=7.4 Hz, 3H, SCH₂CH₂C***H₃***), 1.74 (m, 11H, SCH₂C***H***₂CH₃/ NC(C***H₃***)*₃*), 3.14 (t, J=7.2 Hz, 2H, SC***H₂***CH₂CH₃), 8.54 (s, 1H, 8-***H***).
¹³C NMR (DMSO-*d₆*) δ 13.3 (SCH₂CH₂***C***H₃), 22.1 (SCH₂***C***H₂CH₃), 28.3 (NC(***C***H₃)₃), 32.7 (S***C***H₂CH₂CH₃), 58.0 (NC(***C***H₃)₃), 129.0 (C-5), 144.2 (C-8), 149.3 (C-6), 152.6 (C-4), 162.9 (C-2).

### 9-(tert-Butyl)-N-((1R,2S)-2-(3,4-difluorophenyl)cyclopropyl)-2-(propylthio)-9H-purin-6-amine (9c)

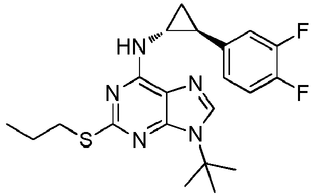

A solution of (**9b**) (143.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 2 h. After distillation of the acetonitrile and triethylamine under vacuum, the residue was purified by silica gel column chromatography.
Yield: 56%.
Melting point: 125-128°C.
¹H NMR (CDCl₃) δ 1.01 (t, J=7.4 Hz, 3H, SCH₂CH₂C***H₃***), 1.29 (m, 2H, NHCH(C***H₂***)CHPh), 1.77 (m, 11H, SCH₂C***H₂***CH₃/NC(C***H₃***)₃), 2.08 (m, 1H, NHCH(CH₂)C***H***Ph), 3.06 (m, 3H, SC***H₂***CH₂CH₃/NHC***H***(CH₂)CHPh), 5.95 (bs, 1H, N***H***), 7.08 (m, 2H, 5'-H/6'-H), 7.17 (m, 1H, 2'-H), 7.70 (s, 1H, 8-***H***).
¹³C NMR (CDCl₃) δ 13.6 (SCH₂CH₂***C***H₃), 15.6 (NHCH(***C***H₂)CHPh), 23.1 (SCH₂***C***H₂CH₃), 25.2
(NHCH(CH₂)***C***HPh), 29.0 (NC(***C***H₃)₃), 33.0 (NH***C***H(CH₂)CHPh), 33.2 (S***C***H₂CH₂CH₃), 57.1 (NC(***C***H₃)₃), 116.2 (d, J=17 Hz, C-2'), 116.9 (d, J=17 Hz, C-5'), 118.9 (C-5), 123.1 (C-6'), 136.5 (C-8), 137.8 (C-1'), 147.9-149.9 (dd, 246 Hz/13 Hz, C-4'), 149.2-151.2 (dd, 247 Hz/13 Hz, C-3'), 150.7 (C-4), 154.9 (C-6), 164.3 (C-2).

### Example 10: synthesis of 9-cyclobutyl-N-((1R,2S)-2-(3,4-difluorophenyl)cyclopropyl)-2-(propylthio)-9H-purin-6-amine (10c)

### 6-Chloro-N⁴-cyclobutyl-2-(propylthio)pyrimidine-4,5-diamine (10a)

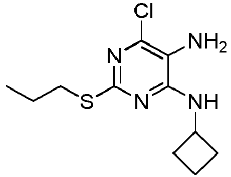

4,6-Dichloro-2-(propylthio)pyrimidin-5-amine (0.5 g, 2.1 mmol) was dissolved in methanol (2 mL) and supplemented with cyclobutylamine (440.0 mg, 6.3 mmol). The reaction mixture was introduced in a sealed vessel and heated at 100°C for 1 h. After concentration of the reaction mixture to dryness under vacuum, the residue was purified by silica gel column chromatography.
Yield: 96%.
Melting point: 73-75.5°C.
¹H NMR (DMSO-*d₆*) δ 0.96 (t, J=7.4 Hz, 3H, SCH₂CH₂C***H₃***), 1.63 (h, J=7.3 Hz, 2H, SCH₂C***H₂***CH₃), 1.72 (m, 2H, NHCH(C***H₂***)*₃*), 1.95 (m, 2H, NHCH(C***H₂***)*₃*), 2.29 (m, 2H, NHCH(C***H₂***)*₃*), 2.94 (t, J=7.2 Hz, 2H, SC***H₂***CH₂CH₃), 4.38 (h, J=8.0 Hz, 1H, NHC***H***(CH₂)₃), 4.80 (s, 2H, *N**H₂***)*,* 7.11 (d, J=6.3 Hz, 1H, N***H***CH(CH₂)₃).
¹³C NMR (DMSO-*d₆*) δ 13.3 (SCH₂CH₂***C***H₃), 14.9 (NHCH(***C***H₂)₃), 22.7 (SCH₂***C***H₂CH₃), 30.2 (NHCH(***C***H₂)₃), 32.1 (S***C***H₂CH₂CH₃), 46.3 (NH***C***H(CH₂)₃), 119.7 (C-5), 137.4 (C-6), 151.5 (C-4), 155.1 (C-2).

### 6-Chloro-9-cyclobutyl-2-(propylthio)-9H-purine (10b)

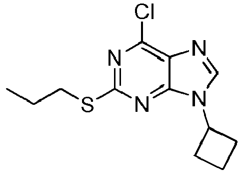

A solution of (**10a**) (273.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography. Yield: 62%.
Melting point: 89-91.5°C.
¹H NMR (DMSO-*d₆*) δ 1.02 (t, J=7.3 Hz, 3H, SCH₂CH₂C***H₃***), 1.75 (h, J=7.3 Hz, 2H, SCH₂C***H₂***CH₃), 1.90 (m, 2H, NCH(C***H₂***)*₃*), 2.47 (m, 2H, NCH(C***H₂***)*₃*), 2.73 (m, 2H, NCH(C***H₂***)*₃*), 3.17 (t, J=7.2 Hz, 2H, SC***H₂***CH₂CH₃), 5.03 (p, J=8.6 Hz, 1H, NC***H***(CH₂)₃), 8.66 (s, 1H, 8-***H***).
¹³C NMR (DMSO-*d₆*) δ 13.3 (SCH₂CH₂***C***H₃), 14.8 (NCH(***C***H₂)₃), 22.1 (SCH₂***C***H₂CH₃), 29.2 (NCH(***C***H₂)₃), 32.7 (S***C***H₂CH₂CH₃), 48.8 (N***C***H(CH₂)₃), 128.3 (C-5), 145.1 (C-8), 148.9 (C-6), 152.5 (C-4), 163.7 (C-2).

### 9-Cyclobutyl-N-((1R,2S)-2-(3,4-difluorophenyl)cyclopropyl)-2-(propylthio)-9H-purin-6-amine (10c)

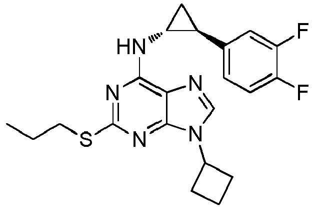

A solution of (**10b**) (142.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 2 h. After distillation of the acetonitrile and triethylamine under vacuum, the residue was purified by silica gel column chromatography.
Yield: 76%.
Melting point: 143-145°C.
¹H NMR (CDCl₃) δ 0.96 (t, J=7.4 Hz, 3H, SCH₂CH₂C***H₃***), 1.32 (m, 2H, NHCH(C***H₂***)CHPh), 1.70 (h, J=7.3 Hz, 2H, SCH₂C***H₂***CH₃), 1.95 (m, 2H, NCH(C***H₂***)₃), 2.09 (m, 1H, NHCH(CH₂)C***H***Ph), 2.56 (m, 2H, NCH(C***H₂***)₃), 2.64 (m, 2H, NCH(C***H₂***)₃), 3.04 (m, 2H, SC***H₂***CH₂CH₃), 3.10 (bs, 1H, NHC***H***(CH₂)CHPh), 4.95 (p, J=8.6 Hz, 1H, NC***H***(CH₂)₃), 6.06 (bs, 1H, N***H***), 7.00 (m, 1H, 6'-H), 7.09 (m, 2H, 2'-H/5'-H), 7.74 (s, 1H, 8-***H***).
¹³C NMR (CDCl₃) δ 13.5 (SCH₂CH₂***C***H₃), 15.3 (NCH(***C***H₂)₃), 16.0 (NHCH(***C***H₂)CHPh), 22.9 (SCH₂***C***H₂CH₃), 25.2 (NHCH(CH₂)***C***HPh), 30.5 (NCH(***C***H₂)₃), 33.2 (S***C***H₂CH₂CH₃), 33.3 (NH***C***H(CH₂)CHPh), 48.8 (N***C***H(CH₂)₃), 115.8 (d, J=17 Hz, C-2'), 116.9 (d, J=17 Hz, C-5'), 117.5 (C-5), 122.8 (C-6'), 137.3 (C-8), 137.8 (C-1'), 147.9-149.9 (dd, 246 Hz/13 Hz, C-4'), 149.2-151.2 (dd, 247 Hz/13 Hz, C-3'), 150.4 (C-4), 154.5 (C-6), 165.2 (C-2).

### Example 11 : synthesis of N-((1R,2S)-2-(3,4-difluorophenyl)cyclopropyl)-9-pentyl-2-(propylthio)-9H-purin-6-amine (11c)

### 6-Chloro-N⁴-pentyl-2-(propylthio)pyrimidine-4,5-diamine (11a)

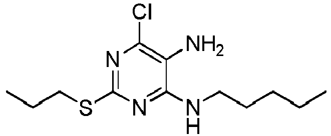

4,6-Dichloro-2-(propylthio)pyrimidin-5-amine (0.5 g, 2.1 mmol) was dissolved in methanol (2 mL) and supplemented with *n*-pentylamine (550.0 mg, 6.3 mmol). The reaction mixture was introduced in a sealed vessel and heated at 100°C for 30 min. After concentration of the reaction mixture to dryness under vacuum, the residue was purified by silica gel column chromatography.
Yield: 96%.
Melting point: 68-69°C.
¹H NMR (DMSO-*d₆*) δ 0.87 (t, J=7.0 Hz, 3H, NHCH₂CH₂CH₂CH₂C***H₃***), 0.95 (t, J=7.4 Hz, 3H, SCH₂CH₂C***H₃***), 1.30 (m, 4H, NHCH₂CH₂C***H₂***C***H₂***CH₃), 1.55 (p, J=7.3 Hz, 2H, NHCH₂C***H₂***CH₂CH₂CH₃), 1.63 (h, J=7.3 Hz, 2H, SCH₂C***H₂***CH₃), 2.94 (t, J=7.2 Hz, 2H, SC***H₂***CH₂CH₃), 3.34 (m, 2H, NHC***H₂***CH₂CH₂CH₂CH₃), 4.75 (s, 2H, N***H₂***), 6.95 (t, J=5.2 Hz, 1H, N***H***CH₂CH₂CH₂CH₂CH₃).
¹³C NMR (DMSO-*d₆*) δ 13.3 (SCH₂CH₂***C***H₃), 13.9 (NHCH₂CH₂CH₂CH₂***C***H₃), 21.9 (NHCH₂CH₂CH₂***C***H₂CH₃), 22.8 (SCH₂***C***H₂CH₃), 28.3 (NHCH₂***C***H₂CH₂CH₂CH₃), 28.7 (NHCH₂CH₂***C***H₂CH₂CH₃), 32.1 (S***C***H₂CH₂CH₃), 40.8 (NH***C***H₂CH₂CH₂CH₂CH₃), 119.8 (C-5), 137.3 (C-6), 152.6 (C-4), 155.2 (C-2).

### 6-Chloro-9-pentyl-2-(propylthio)-9H-purine (11b)

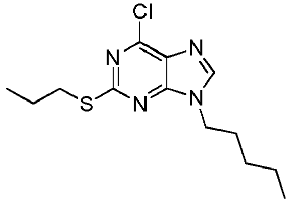

A solution of (**11a**) (289.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography. Yield: 87%.
Melting point: liquid.
¹H NMR (DMSO-*d₆*) δ 0.85 (t, J=7.3 Hz, 3H, NCH₂CH₂CH₂CH₂C***H₃***), 1.02 (t, J=7.4 Hz, 3H, SCH₂CH₂C***H₃***), 1.23 (m, 2H, NCH₂CH₂C***H₂***CH₂CH₃), 1.31 (m, 2H, NCH₂CH₂CH₂C***H₂***CH₃), 1.75 (h, J=7.3 Hz, 2H, SCH₂C***H₂***CH₃), 1.86 (p, J=7.2 Hz, 2H, NCH₂C***H₂***CH₂CH₂CH₃), 3.17 (t, J=7.2 Hz, 2H, SC***H₂***CH₂CH₃), 4.22 (t, J=7.1 Hz, 2H, NC***H₂***CH₂CH₂CH₂CH₃), 8.56 (s, 1H, 8-***H***).
¹³C NMR (DMSO-*d₆*) δ 13.3 (SCH₂CH₂***C***H₃), 13.7 (NCH₂CH₂CH₂CH₂***C***H₃), 21.5 (NCH₂CH₂CH₂***C***H₂CH₃), 22.1 (SCH₂***C***H₂CH₃), 28.1 (NCH₂CH₂***C***H₂CH₂CH₃), 28.5 (NCH₂***C***H₂CH₂CH₂CH₃), 32.6 (S***C***H₂CH₂CH₃), 43.6 (N***C***H₂CH₂CH₂CH₂CH₃), 127.9 (C-5), 146.3 (C-8), 148.9 (C-6), 152.8 (C-4), 163.8 (C-2).

### N-((1R,2S)-2-(3,4-Difluorophenyl)cyclopropyl)-9-pentyl-2-(propylthio)-9H-purin-6-amine (11c)

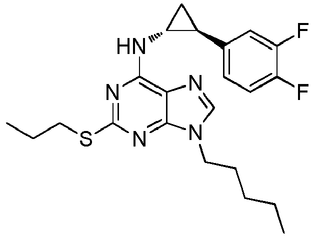

A solution of (**11b**) (150.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 3 h. After distillation of the acetonitrile and triethylamine under vacuum, the residue was purified by silica gel column chromatography.
Yield: 83%.
Melting point: 98-100.5°C.
¹H NMR (CDCl₃) δ 0.89 (t, J=7.2 Hz, 3H, NCH₂CH₂CH₂CH₂C***H₃***), 0.95 (t, J=7.4 Hz, 3H, SCH₂CH₂C***H₃***), 1.32 (m, 6H, NCH₂CH₂C***H₂***C***H₂***CH_{3/}NHCH(C***H₂***)CHPh), 1.68 (h, J=7.3 Hz, 2H, SCH₂C***H₂***CH₃), 1.87 (p, J=7.3 Hz, 2H, NCH₂C***H₂***CH₂CH₂CH₃), 2.09 (m, 1H, NHCH(CH₂)C***H***Ph), 3.04 (m, 2H, SC***H₂***CH₂CH₃), 3.10 (bs, 1H, NHC***H***(CH₂)CHPh), 4.12 (t, J=7.2 Hz, 2H, NC***H₂***CH₂CH₂CH₂CH₃), 5.97 (bs, 1H, N***H***), 7.00 (m, 1H, 6'-H), 7.09 (m, 2H, 2'-H/5'-H), 7.60 (s, 1H, 8-***H***).
¹³C NMR (CDCl₃) δ 13.5 (SCH₂CH₂***C***H₃), 13.9 (NCH₂CH₂CH₂CH₂***C***H₃), 16.0 (NHCH(***C***H₂)CHPh), 22.2 (NCH₂CH₂CH₂***C***H₂CH₃), 22.9 (SCH₂***C***H₂CH₃), 25.2 (NHCH(CH₂)***C***HPh), 28.7 (NCH₂CH₂***C***H₂CH₂CH₃), 29.7 (NCH₂***C***H₂CH₂CH₂CH₃), 33.2 (S***C***H₂CH₂CH₃), 33.3 (NH***C***H(CH₂)CHPh), 43.7 (N***C***H₂CH₂CH₂CH₂CH₃), 115.7 (d, J=17 Hz, C-2'), 116.9 (d, J=17 Hz, C-5'), 117.5 (C-5), 122.7 (C-6'), 137.9 (C-1'), 139.0 (C-8), 147.9-149.9 (dd, 246 Hz/13 Hz, C-4'), 149.2-151.2 (dd, 247 Hz/13 Hz, C-3'), 150.5 (C-4), 154.6 (C-6), 165.4 (C-2).

### Example 12 : synthesis of 9-cyclopentyl-N-((1R,2S)-2-(3,4-difluorophenyl)cyclopropyl)-2-(propylthio)-9H-purin-6-amine (12c)

### 6-Chloro-N⁴-cyclopentyl-2-(propylthio)pyrimidine-4,5-diamine (12a)

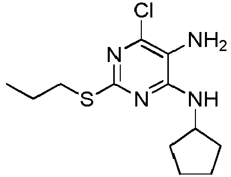

4,6-Dichloro-2-(propylthio)pyrimidin-5-amine (0.5 g, 2.1 mmol) was dissolved in methanol (2 mL) and supplemented with cyclopentylamine (536.0 mg, 6.3 mmol). The reaction mixture was introduced in a sealed vessel and heated at 100°C for 2 h. After concentration of the reaction mixture to dryness under vacuum, the residue was purified by silica gel column chromatography. Yield: 95%.
Melting point: 86-88°C.
¹H NMR (DMSO-*d₆*) δ 0.95 (t, J=7.4 Hz, 3H, SCH₂CH₂C***H₃***), 1.49 (m, 2H, NHCH(C***H₂***)*₄*), 1.55 (m, 2H, NHCH(C***H₂***)*₄*), 1.64 (m, 2H, SCH₂C***H₂***CH₃), 1.70 (m, 2H, NHCH(C***H₂***)*₄*), 1.96 (m, 2H, NHCH(C***H₂***)*₄*), 2.94 (t, J=7.2 Hz, 2H, SC***H₂***CH₂CH₃), 4.25 (h, J=6.7 Hz, 1H, NHC***H***(CH₂)₄), 4.83 (s, 2H, N***H₂***), 6.76 (d, J=6.3 Hz, 1H, N***H***CH(CH₂)*₄*).
¹³C NMR (DMSO-*d₆*) δ 13.3 (SCH₂CH₂***C***H₃), 22.9 (SCH₂***C***H₂CH₃), 23.5 (NHCH(***C***H₂)*₄*), 32.1 (S***C***H₂CH₂CH₃), 32.2 (NHCH(***C***H₂)*₄*), 52.7 (NH***C***H(CH₂)*₄*), 119.9 (C-5), 137.2 (C-6), 152.0 (C-4), 155.0 (C-2).

### 6-Chloro-9-cyclopentyl-2-(propylthio)-9H-purine (12b)

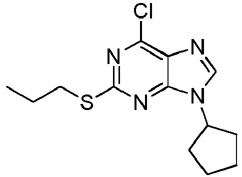

A solution of (**12a**) (287.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography.
Yield: 37%.
Melting point: 81-83°C.
¹H NMR (DMSO-*d₆*) δ 1.01 (t, J=7.3 Hz, 3H, SCH₂CH₂C***H₃***), 1.74 (m, 4H, SCH₂C***H₂***CH₃/NCH(C***H₂***)*₄*), 1.90 (m, 2H, NCH(C***H₂***)*₄*), 2.06 (m, 2H, NCH(C***H₂***)*₄*), 2.19 (m, 2H, NCH(C***H₂***)*₄*), 3.16 (t, J=7.2 Hz, 2H, SC***H₂***CH₂CH₃), 4.90 (p, J=7.6 Hz, 1H, NC***H***(CH₂)₄), 8.59 (s, 1H, 8-***H***).
¹³C NMR (DMSO-*d₆*) δ 13.3 (SCH₂CH₂***C***H₃), 14.8 (NCH(***C***H₂)₄), 22.1 (SCH₂***C***H₂CH₃), 29.2 (NCH(***C***H₂)₄), 32.7 (S***C***H₂CH₂CH₃), 48.8 (N***C***H(CH₂)₄), 128.3 (C-5), 145.1 (C-8), 148.9 (C-6), 152.5 (C-4), 163.7 (C-2).

### 9-Cyclopentyl-N-((1R,2S)-2-(3,4-difluorophenyl)cyclopropyl)-2-(propylthio)-9H-purin-6-amine (12c)

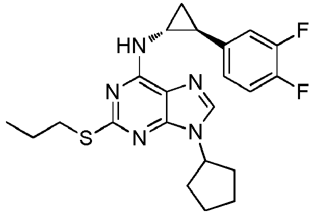

A solution of (**12b**) (149.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 3 h. After distillation of the acetonitrile and triethylamine under vacuum, the residue was purified by silica gel column chromatography.
Yield: 35%.
Melting point: 112-114°C.
¹H NMR (CDCl₃) δ 0.95 (t, J=7.4 Hz, 3H, SCH₂CH₂C***H₃***), 1.31 (m, 2H, NHCH(C***H₂***)CHPh), 1.68 (h, J=7.4 Hz, 2H, SCH₂C***H₂***CH₃), 1.79 (m, 2H, NCH(C***H₂***)₄), 1.94 (m, 2H, NCH(C***H₂***)₄), 1.99 (m, 2H, NCH(C***H₂***)₄), 2.09 (m, 1H, NHCH(CH₂)C***H***Ph), 2.26 (m, 2H, NCH(C***H₂***)₄), 3.04 (m, 2H, SC***H₂***CH₂CH₃), 3.10 (bs, 1H, NHC***H***(CH₂)CHPh), 4.84 (p, J=7.4 Hz, 1H, NC***H***(CH₂)₄), 5.92 (bs, 1H, N***H***), 7.00 (m, 1H, 6'-H), 7.09 (m, 2H, 2'-H/5'-H), 7.65 (s, 1H, 8-***H***).
¹³C NMR (CDCl₃) δ 13.5 (SCH₂CH₂***C***H₃), 16.0 (NHCH(***C***H₂)CHPh), 22.9 (SCH₂***C***H₂CH₃), 24.1 (NCH(***C***H₂)₄), 25.3 (NHCH(CH₂)***C***HPh), 32.6 (NCH(***C***H₂)₄), 33.2 (S***C***H₂CH₂CH₃), 33.3 (NH***C***H(CH₂)CHPh), 56.0 (N***C***H(CH₂)₄), 115.7 (d, J=17 Hz, C-2'), 116.9 (d, J=17 Hz, C-5'), 117.9 (C-5), 122.8 (C-6'), 137.4 (C-8), 137.9 (C-1'), 147.9-149.9 (dd, 246 Hz/13 Hz, C-4'), 149.2-151.2 (dd, 247 Hz/13 Hz, C-3'), 150.0 (C-4), 154.6 (C-6), 165.0 (C-2).

### Example 13: synthesis of N-((1R,2S)-2-(3,4-difluorophenyl)cyclopropyl)-9-hexyl-2-(propylthio)-9H-purin-6-amine (13c)

### 6-Chloro-N⁴-hexyl-2-(propylthio)pyrimidine-4,5-diamine (13a)

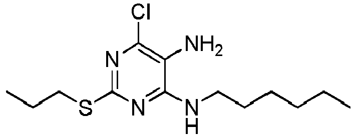

4,6-Dichloro-2-(propylthio)pyrimidin-5-amine (0.5 g, 2.1 mmol) was dissolved in methanol (2 mL) and supplemented with *n*-hexylamine (638.0 mg, 6.3 mmol). The reaction mixture was introduced in a sealed vessel and heated at 100°C for 1 h. After concentration of the reaction mixture to dryness under vacuum, the residue was purified by silica gel column chromatography.
Yield: 85%.
Melting point: 54-57°C.
¹H NMR (DMSO-*d₆*) δ 0.87 (t, J=6.4 Hz, 3H, NHCH₂CH₂CH₂CH₂CH₂C***H₃***), 0.95 (t, J=7.3 Hz, 3H, SCH₂CH₂C***H₃***), 1.29 (m, 6H, NHCH₂CH₂C***H₂***C***H₂***C***H₂***CH₃), 1.54 (p, J=6.9 Hz, 2H, NHCH₂C***H₂***CH₂CH₂CH₂CH₃), 1.63 (h, J=7.3 Hz, 2H, SCH₂C***H₂***CH₃), 2.94 (t, J=7.2 Hz, 2H, SC***H₂***CH₂CH₃), 3.34 (m, 2H, NHC***H₂***CH₂CH₂CH₂CH₂CH₃), 4.76 (s, 2H, N***H₂***), 6.95 (t, J=4.9 Hz, 1H, N***H***CH₂CH₂CH₂CH₂CH₂CH₃).
¹³C NMR (DMSO-*d₆*) δ 13.3 (SCH₂CH₂***C***H₃), 13.9(NHCH₂CH₂CH₂CH₂CH₂***C***H₃), 22.1 (NHCH₂CH₂CH₂CH₂***C***H₂CH₃), 22.8 (SCH₂***C***H₂CH₃), 26.1 (NHCH₂CH₂CH₂***C***H₂CH₂CH₃), 28.6 (NHCH₂***C***H₂CH₂CH₂CH₂CH₃), 31.0 (NHCH₂CH₂***C***H₂CH₂CH₂CH₃), 32.1 (S***C***H₂CH₂CH₃), 40.9 (NH***C***H₂CH₂CH₂CH₂CH₂CH₃), 119.8 (C-5), 137.3 (C-6), 152.6 (C-4), 155.2 (C-2).

### 6-Chloro-9-hexyl-2-(propylthio)-9H-purine (13b)

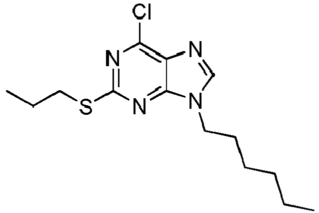

A solution of (**13a**) (303.0 mg, 1 mmol) in acetic acid (2.5 mL) and triethyl orthoformate (2.5 mL, 15 mmol) was heated at a temperature of 130°C under reflux for 1 h. After distillation of the acid acetic and triethyl orthoformate under vacuum, the residue was purified by silica gel column chromatography.
Yield: 90%.
Melting point: liquid.
¹H NMR (DMSO-*d₆*) δ 0.83 (t, J=6.8 Hz, 3H, NCH₂CH₂CH₂CH₂CH₂C***H₃***), 1.01 (t, J=7.4 Hz, 3H, SCH₂CH₂C***H***₃), 1.25 (m, 6H, NCH₂CH₂C***H₂***C***H₂***C***H₂***CH₃), 1.74 (h, J=7.3 Hz, 2H, SCH₂C***H₂***CH₃), 1.84 (p, J=7.2 Hz, 2H, NCH₂C***H₂***CH₂CH₂CH₂CH₃), 3.16 (t, J=7.2 Hz, 2H, SC***H₂***CH₂CH₃), 4.21 (t, J=7.1 Hz, 2H, NC***H₂***CH₂CH₂CH₂CH₂CH₃), 8.56 (s, 1H, 8-***H***).
¹³C NMR (DMSO-*d₆*) δ 13.3 (SCH₂CH₂***C***H₃), 13.8 (NCH₂CH₂CH₂CH₂CH₂***C***H₃), 21.9 (NCH₂CH₂CH₂CH₂***C***H₂CH₃), 22.1 (SCH₂***C***H₂CH₃), 25.5 (NCH₂CH₂***C***H₂CH₂CH₂CH₃), 28.8 (NCH₂***C***H₂CH₂CH₂CH₂CH₃), 30.5 (NCH₂CH₂CH₂***C***H₂CH₂CH₃), 32.6 (S***C***H₂CH₂CH₃), 43.6 (N***C***H₂CH₂CH₂CH₂CH₂CH₃), 127.9 (C-5), 146.3 (C-8), 148.9 (C-6), 152.8 (C-4), 163.8 (C-2).

### N-((1R,2S)-2-(3,4-Difluorophenyl)cyclopropyl)-9-hexyl-2-(propylthio)-9H-purin-6-amine (13c)

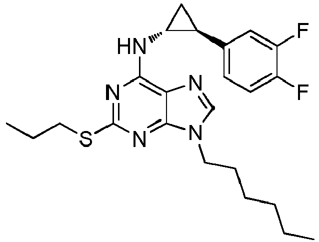

A solution of (**13b**) (157.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 60%.
Melting point: 84-86°C.
¹H NMR (CDCl₃) δ 0.88 (m, 3H, NCH₂CH₂CH₂CH₂CH₂C***H₃***), 0.95 (t, J=7.4 Hz, 3H, SCH₂CH₂C***H₃***), 1.30 (m, 8H, NCH₂CH₂C***H₂***C***H₂***C***H₂***CH₃/NHCH(C***H₂***)CHPh), 1.68 (h, J=7.3 Hz, 2H, SCH₂C***H₂***CH₃), 1.86 (m, 2H, NCH₂C***H₂***CH₂CH₂CH₂CH₃), 2.09 (m, 1H, NHCH(CH₂)C***H***Ph), 3.03 (m, 2H, SC***H₂***CH₂CH₃), 3.10 (bs, 1H, NHC***H***(CH₂)CHPh), 4.12 (t, J=7.2 Hz, 2H, NC***H₂***CH₂CH₂CH₂CH₂CH₃), 5.96 (bs, 1H, N***H***), 6.99 (m, 1H, 6'-H), 7.09 (m, 2H, 2'-H/5'-H), 7.60 (s, 1H, 8-***H***).
¹³C NMR (CDCl₃) δ 13.5 (SCH₂CH₂***C***H₃), 14.0 (NCH₂CH₂CH₂CH₂CH₂***C***H₃), 16.0 (NHCH(***C***H₂)CHPh), 22.5 (NCH₂CH₂CH₂CH₂***C***H₂CH₃), 22.9 (SCH₂***C***H₂CH₃), 25.2 (NHCH(CH₂)***C***HPh), 26.3 (NCH₂CH₂***C***H₂CH₂CH₂CH₃), 29.9 (NCH₂***C***H₂CH₂CH₂CH₂CH₃), 31.2 (NCH₂CH₂CH₂***C***H₂CH₂CH₃), 33.2 (S***C***H₂CH₂CH₃), 33.3 (NH***C***H(CH₂)CHPh), 43.7 (N***C***H₂CH₂CH₂CH₂CH₂CH₃), 115.7 (d, J=17 Hz, C-2'), 116.9 (d, J=17 Hz, C-5'), 117.5 (C-5), 122.7 (C-6'), 137.9 (C-1'), 139.0 (C-8), 147.9-149.9 (dd, 246 Hz/13 Hz, C-4'), 149.2-151.2 (dd, 247 Hz/13 Hz, C-3'), 150.5 (C-4), 154.6 (C-6), 165.4 (C-2).

### Example 14: synthesis of 9-cyclohexyl-N-((1R,2S)-2-(3,4-difluorophenyl)cyclopropyl)-2-(propylthio)-9H-purin-6-amine (14c)

### 6-Chloro-N⁴-cyclohexyl-2-(propylthio)pyrimidine-4,5-diamine (14a)

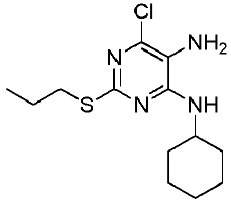

4,6-Dichloro-2-(propylthio)pyrimidin-5-amine (0.5 g, 2.1 mmol) was dissolved in methanol (2 mL) and supplemented with cyclohexylamine (625.0 mg, 6.3 mmol). The reaction mixture was introduced in a sealed vessel and heated at 100°C for 1 h. After concentration of the reaction mixture to dryness under vacuum, the residue was purified by silica gel column chromatography.
Yield: 91%.
Melting point: 90-93°C.
¹H NMR (DMSO-*d₆*) δ 0.96 (t, J=7.4 Hz, 3H, SCH₂CH₂C***H₃***), 1.22 (m, 5H, NHCH(C***H₂***)*₅*), 1.64 (m, 3H, SCH₂C***H₂***CH₃/NHCH(C***H₂***)*₅*), 1.75 (m, 2H, NHCH(C***H₂***)*₅*), 1.93 (m, 2H, NHCH(C***H₂***)*₅*), 2.92 (t, J=7.3 Hz, 2H, SC***H₂***CH₂CH₃), 3.84 (m, 1H, NHC***H***(CH₂)₅), 4.82 (s, 2H, N***H₂***), 6.68 (d, J=7.1 Hz, 1H, N***H***CH(CH₂)*₅*).
¹³C NMR (DMSO-*d₆*) δ 13.4 (SCH₂CH₂***C***H₃), 23.0 (SCH₂***C***H₂CH₃), 24.8 (NHCH(***C***H₂)*₅*), 25.3 (NHCH(***C***H₂)*₅*), 32.1 (S***C***H₂CH₂CH₃), 32.3 (NHCH(***C***H₂)*₅*), 49.9 (NH***C***H(CH₂)*₅*), 119.7 (C-5), 137.4 (C-6), 151.6 (C-4), 155.0 (C-2).

### 6-Chloro-9-cyclohexyl-2-(propylthio)-9H-purine (14b)

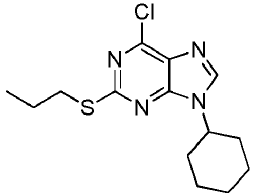

A solution of (**14a**) (301.0 mg, 1 mmol) in acetic acid (2.5 mL) and triethyl orthoformate (2.5 mL, 15 mmol) was heated at a temperature of 130°C under reflux for 2 h. After distillation of the acid acetic and triethyl orthoformate under vacuum, the residue was purified by silica gel column chromatography.
Yield: 62%.
Melting point: 93-95°C.
¹H NMR (DMSO-*d₆*) δ 1.02 (t, J=7.3 Hz, 3H, SCH₂CH₂C***H₃***), 1.25 (m, 1H, NCH(C***H₂***)*₅*), 1.44 (m, 2H, NCH(C***H₂***)*₅*), 1.75 (m, 3H, SCH₂C***H₂***CH₃/NCH(C***H₂***)*₅*), 1.87 (m, 2H, NCH(C***H₂***)*₅*), 1.99 (m, 4H, NCH(C***H₂***)*₅*), 3.15 (t, J=7.2 Hz, 2H, SC***H₂***CH₂CH₃), 4.41 (m, 1H, NC***H***(CH₂)₅), 8.61 (s, 1H, 8-***H***).
¹³C NMR (DMSO-*d₆*) δ 13.3 (SCH₂CH₂***C***H₃), 22.2 (SCH₂***C***H₂CH₃), 24.8 (NCH(***C***H₂)₅), 25.0 (NCH(***C***H₂)₅), 31.7 (NCH(***C***H₂)₅), 32.7 (S***C***H₂CH₂CH₃), 55.0 (N***C***H(CH₂)₅), 128.3 (C-5), 144.8 (C-8), 149.0 (C-6), 152.3 (C-4), 163.5 (C-2).

### 9-Cyclohexyl-N-((1R,2S)-2-(3,4-difluorophenyl)cyclopropyl)-2-(propylthio)-9H-purin-6-amine (14c)

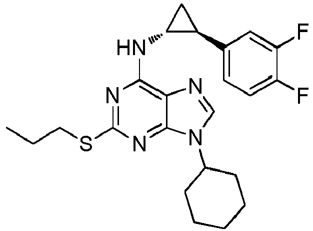

A solution of (**14b**) (156.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 84%.
Melting point: 85-88°C.
¹H NMR (CDCl₃) δ 0.94 (t, J=7.4 Hz, 3H, SCH₂CH₂C***H₃***), 1.30 (m, 3H, NHCH(C***H₂***)CHPh/NCH(C***H₂***)₅), 1.49 (m, 2H, NCH(C***H₂***)₅), 1.68 (h, J=7.3 Hz, 2H, SCH₂C***H₂***CH₃), 1.79 (m, 3H, NCH(C***H₂***)₅) 1.92 (m, 2H, NCH(C***H₂***)₅), 2.08 (m, 1H, NHCH(CH₂)C***H***Ph), 2.14 (m, 2H, NCH(C***H₂***)₅), 3.03 (m, 2H, SC***H₂***CH₂CH₃), 3.11 (bs, 1H, NHC***H***(CH₂)CHPh), 4.36 (m, 1H, NC***H***(CH₂)₅), 5.98 (bs, 1H, N***H***), 6.99 (m, 1H, 6'-H), 7.08 (m, 2H, 2'-H/5'-H), 7.67 (s, 1H, 8-***H***).
¹³C NMR (CDCl₃) δ 13.5 (SCH₂CH₂***C***H₃), 16.1 (NHCH(***C***H₂)CHPh), 23.0 (SCH₂***C***H₂CH₃), 25.2
(NHCH(CH₂)***C***HPh), 25.3 (NCH(***C***H₂)₅), 25.6 (NCH(***C***H₂)₅), 33.2 (S***C***H₂CH₂CH₃), 33.3 (NH***C***H(CH₂)CHPh), 54.2 (N***C***H(CH₂)₅), 115.6 (d, J=17 Hz, C-2'), 116.9 (d, J=17 Hz, C-5'), 117.7 (C-5), 122.7 (C-6'), 137.0 (C-8), 138.0 (C-1'), 147.9-149.9 (dd, 246 Hz/13 Hz, C-4'), 149.2-151.2 (dd, 247 Hz/13 Hz, C-3'), 150.5 (C-4), 154.6 (C-6), 165.0 (C-2).

### Example 15: synthesis of 9-allyl-N-((1R,2S)-2-(3,4-difluorophenyl)cyclopropyl)-2-(propylthio)-9H-purin-6-amine (15c)

### N⁴-Allyl-6-chloro-2-(propylthio)pyrimidine-4,5-diamine (15a)

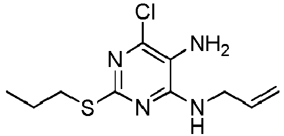

4,6-Dichloro-2-(propylthio)pyrimidin-5-amine (0.5 g, 2.1 mmol) was dissolved in methanol (2 mL) and supplemented with allylamine (360.0 mg, 6.3 mmol). The reaction mixture was introduced in a sealed vessel and heated at 100°C for 30 min. After concentration of the reaction mixture to dryness under vacuum, the residue was purified by silica gel column chromatography.
Yield: 92%.
Melting point: 55-57°C.
¹H NMR (DMSO-*d₆*) δ 0.94 (t, J=7.4 Hz, 3H, SCH₂CH₂C***H₃***), 1.62 (h, J=7.3 Hz, 2H, SCH₂C***H₂***CH₃), 2.93 (t, J=7.2 Hz, 2H, SC***H₂***CH₂CH₃), 4.02 (tt, J=5.4 Hz/1.5 Hz, 2H, NHC***H₂***CHCH₂), 4.80 (s, 2H, N***H₂***), 5.11 (dq, J=10.3 Hz/1.4 Hz, 1H, NHCH₂CHC***H₂***), 5.18 (dq, J=17.2 Hz/1.5 Hz, 1H, NHCH₂CHC***H₂***), 5.92 (ddt, J=17.1 Hz/10.4 Hz/5.3 Hz, 1H, NHCH₂C***H***CH₂), 7.15 (t, J=5.4 Hz, 1H, N***H***CH₂CHCH₂).
¹³C NMR (DMSO-*d₆*) δ 13.3 (SCH₂CH₂***C***H₃), 22.7 (SCH₂***C***H₂CH₃), 32.1 (S***C***H₂CH₂CH₃), 43.1 (NH***C***H₂CHCH₂), 115.6 (NHCH₂CH***C***H₂), 120.0 (C-5), 135.0 (NHCH₂***C***HCH₂), 137.5 (C-6), 152.3 (C-4), 155.2 (C-2).

### 9-allyl-6-chloro-2-(propylthio)-9H-purine (15b)

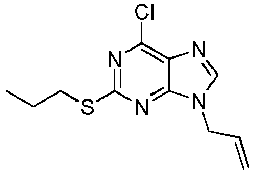

A solution of (**15a**) (259.0 mg, 1 mmol) in acetic acid (2.5 mL) and triethyl orthoformate (2.5 mL, 15 mmol) was heated at a temperature of 130°C under reflux for 2 h. After distillation of the acid acetic and triethyl orthoformate under vacuum, the residue was purified by silica gel column chromatography. Yield: 89%.
Melting point: 47.5-49.5°C.
¹H NMR (DMSO-*d₆*) δ 1.00 (t, J=7.4 Hz, 3H, SCH₂CH₂C***H₃***), 1.73 (h, J=7.3 Hz, 2H, SCH₂C***H₂***CH₃), 3.15 (t, J=7.2 Hz, 2H, SC***H₂***CH₂CH₃), 4.87 (dt, J=5.5 Hz/1.3 Hz, 2H, NC***H₂***CHCH₂), 5.13 (dd, J=17.1 Hz/1.3 Hz, 1H, NCH₂CHC***H₂***), 5.24 (dd, J=10.3 Hz/1.3 Hz, 1H, NCH₂CHC***H₂***), 6.07 (m, 1H, NCH₂C***H***CH₂), 8.52 (s, 1H, 8-***H***). ¹³C NMR (DMSO-*d₆*) δ 13.2 (SCH₂CH₂***C***H₃), 22.0 (SCH₂***C***H₂CH₃), 32.6 (S***C***H₂CH₂CH₃), 45.7 (N***C***H₂CHCH₂), 118.2 (NCH₂CH***C***H₂), 127.9 (C-5), 132.4 (NCH₂***C***HCH₂), 146.2 (C-8), 149.0 (C-6), 152.7 (C-4), 164.1 (C-2).

### 9-Allyl-N-((1R,2S)-2-(3,4-difluorophenyl)cyclopropyl)-2-(propylthio)-9H-purin-6-amine (15c)

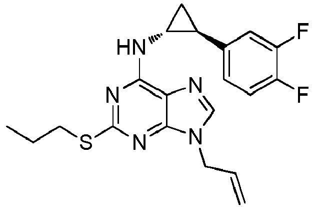

A solution of (**15b**) (135.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 89%.
Melting point: liquid.
¹H NMR (CDCl₃) δ 0.94 (t, J=7.4 Hz, 3H, SCH₂CH₂C***H₃***), 1.32 (m, 2H, NHCH(C***H₂***)CHPh), 1.66 (h, J=7.2 Hz, 2H, SCH₂C***H₂***CH₃), 2.09 (m, 1H, NHCH(CH₂)C***H***Ph), 3.04 (m, 2H, SC***H₂***CH₂CH₃), 3.12 (bs, 1H, NHC***H***(CH₂)CHPh), 4.74 (d, J=5.9 Hz, 2H, NC***H₂***CHCH₂), 5.23 (dd, J=17.1 Hz/1.0 Hz, 1H, NCH₂CHC***H₂***), 5.30 (dd, J=10.2 Hz/1.0 Hz, 1H, NCH₂CHC***H₂***), 6.01 (m, 2H, NCH₂C***H***CH₂/N***H***), 6.99 (m, 1H, 6'-H), 7.08 (m, 2H, 2'-H/5'-H), 7.62 (s, 1H, 8-***H***).
¹³C NMR (CDCl₃) δ 13.4 (SCH₂CH₂***C***H₃), 16.1 (NHCH(***C***H₂)CHPh), 22.8 (SCH₂***C***H₂CH₃), 25.2 (NHCH(CH₂)***C***HPh), 33.2 (S***C***H₂CH₂CH₃), 33.3 (NH***C***H(CH₂)CHPh), 45.6 (N***C***H₂CHCH₂), 115.6 (d, J=17 Hz, C-2'), 116.9 (d, J=17 Hz, C-5'), 117.3 (C-5), 119.0 (NCH₂CH***C***H₂), 122.7 (C-6'), 132.0 (NCH₂***C***HCH₂), 137.9 (C-1'), 138.7 (C-8),147.9-149.9 (dd, 246 Hz/13 Hz, C-4'), 149.2-151.2 (dd, 247 Hz/13 Hz, C-3'), 150.3 (C-4), 154.6 (C-6), 165.7 (C-2).

### Example 16: synthesis of 2-(6-(((1R,2S)-2-(3,4-difluorophenyl)cyclopropyl)amino)-2-(propylthio)-9H-purin-9-yl)ethanol (16c)

### 2-((5-Amino-6-chloro-2-(propylthio)pyrimidin-4-yl)amino)ethanol (16a)

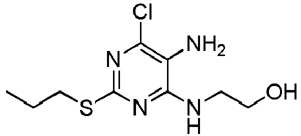

4,6-Dichloro-2-(propylthio)pyrimidin-5-amine (0.5 g, 2.1 mmol) was dissolved in methanol (2 mL) and supplemented with 2-aminoethanol (385.0 mg, 6.3 mmol). The reaction mixture was introduced in a sealed vessel and heated at 100°C for 30 min. After concentration of the reaction mixture to dryness under vacuum, the residue was purified by silica gel column chromatography.
Yield: 79%.
Melting point: 99-102°C.
¹H NMR (DMSO-*d₆*) δ 0.95 (t, J=7.4 Hz, 3H, SCH₂CH₂C***H₃***), 1.63 (h, J=7.3 Hz, 2H, SCH₂C***H₂***CH₃), 2.93 (t, J=7.2 Hz, 2H, SC***H₂***CH₂CH₃), 3.43 (d, J=5.7 Hz, 2H, NHC***H₂***CH₂OH), 3.55 (d, J=5.7 Hz, 2H, NHCH₂C***H₂***OH), 4.78 (t, J=5.5 Hz, 1H, NHCH₂CH₂O***H***), 4.80 (s, 2H, N***H₂***), 7.03 (t, J=5.2 Hz, 1H, N***H***CH₂CH₂OH).
¹³C NMR (DMSO-*d₆*) δ 13.3 (SCH₂CH₂***C***H₃), 22.7 (SCH₂***C***H₂CH₃), 32.1 (S***C***H₂CH₂CH₃), 43.7 (NH***C***H₂CH₂OH), 59.2 (NHCH₂***C***H₂OH), 120.0 (C-5), 137.4 (C-6), 152.7 (C-4), 155.1 (C-2).

### 2-(6-Chloro-2-(propylthio)-9H-purin-9-yl)ethanol (16b)

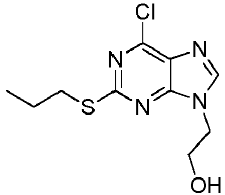

A solution of (**16a**) (263.0 mg, 1 mmol) in acetic acid (2.5 mL) and triethyl orthoformate (2.5 mL, 15 mmol) was heated at a temperature of 130°C under reflux for 4 h. After distillation of the acid acetic and triethyl orthoformate under vacuum, the residue was purified by silica gel column chromatography.
Yield: 57%.
Melting point: 81-83°C.
¹H NMR (DMSO-*d₆*) δ 1.01 (t, J=7.4 Hz, 3H, SCH₂CH₂C***H₃***), 1.73 (h, J=7.4 Hz, 2H, SCH₂C***H₂***CH₃), 3.17 (t, J=7.2 Hz, 2H, SC***H₂***CH₂CH₃), 3.78 (q, J=5.4 Hz, 2H, NCH₂C***H₂***OH), 4.26 (t, J=5.4 Hz, 2H, NC***H₂***CH₂OH), 4.99 (t, J=5.6 Hz, 1H, O***H***), 8.48 (s, 1H, 8-***H***).
¹³C NMR (DMSO-*d₆*) δ 13.2 (SCH₂CH₂***C***H₃), 22.0 (SCH₂***C***H₂CH₃), 32.6 (S***C***H₂CH₂CH₃), 46.5 (N***C***H₂CH₂OH), 58.7 (NCH₂***C***H₂OH), 128.0 (C-5), 146.8 (C-8), 148.7 (C-6), 153.0 (C-4), 163.7 (C-2).

### 2-(6-(((1R,2S)-2-(3,4-Difluorophenyl)cyclopropyl)amino)-2-(propylthio)-9H-purin-9-yl)ethanol (16c)

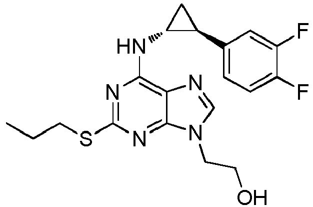

A solution of (**16b**) (137.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 42%.
Melting point: 81-83°C.
¹H NMR (CDCl₃) δ 0.95 (t, J=7.4 Hz, 3H, SCH₂CH₂C***H₃***), 1.32 (m, 2H, NHCH(C***H₂***)CHPh), 1.66 (bh, J=7.3 Hz, 3H, O***H***/SCH₂C***H₂***CH₃), 2.09 (m, 1H, NHCH(CH₂)C***H***Ph), 3.01 (m, 2H, SC***H₂***CH₂CH₃), 3.11 (bs, 1H, NHC***H***(CH₂)CHPh), 4.02 (m, 2H, NCH₂C***H₂***OH), 4.29 (m, 2H, NC***H₂***CH₂OH), 6.05 (bs, 1H, N***H***), 7.00 (m, 1H, 6'-H), 7.09 (m, 2H, 2'-H/5'-H), 7.61 (s, 1H, 8-***H***).
¹³C NMR (CDCl₃) δ 13.4 (SCH₂CH₂***C***H₃), 15.9 (NHCH(***C***H₂)CHPh), 22.6 (SCH₂***C***H₂CH₃), 25.2 (NHCH(CH₂)***C***HPh), 33.1 (S***C***H₂CH₂CH₃), 33.2 (NH***C***H(CH₂)CHPh), 48.2 (N***C***H₂CH₂OH), 61.6 (NCH₂***C***H₂OH), 115.7 (d, J=17 Hz, C-2'), 117.0 (d, J=17 Hz, C-5'), 117.7 (C-5), 122.7 (C-6'), 137.7 (C-1'), 139.7 (C-8), 147.9-149.9 (dd, 246 Hz/13 Hz, C-4'), 149.2-151.2 (dd, 247 Hz/13 Hz, C-3'), 150.1 (C-4), 154.6 (C-6), 165.8 (C-2).

### Example 17: synthesis of N-((1R,2S)-2-(3,4-difluorophenyl)cyclopropyl)-9-(prop-2-yn-1-yl)-2-(propylthio)-9H-purin-6-amine hydrochloride (17c.HCl)

### 6-Chloro-N⁴-(prop-2-yn-1-yl)-2-(propylthio)pyrimidine-4,5-diamine (17a)

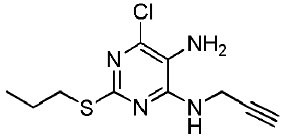

4,6-Dichloro-2-(propylthio)pyrimidin-5-amine (0.5 g, 2.1 mmol) was dissolved in methanol (2 mL) and supplemented with propargylamine (347.0 mg, 6.3 mmol). The reaction mixture was introduced in a sealed vessel and heated at 100°C for 3 h. After concentration of the reaction mixture to dryness under vacuum, the residue was purified by silica gel column chromatography.
Yield: 78%.
Melting point: 90-92°C.
¹H NMR (DMSO-*d₆*) δ 0.96 (t, J=7.4 Hz, 3H, SCH₂CH₂C***H₃***), 1.66 (h, J=7.3 Hz, 2H, SCH₂C***H₂***CH₃), 2.97 (t, J=7.2 Hz, 2H, SC***H₂***CH₂CH₃), 3.15 (t, J=2.4 Hz, 1H, NHCH₂CC***H***), 4.16 (dd, J=4.8 Hz/2.3 Hz, 2H, NHC***H₂***CCH), 4.83 (s, 2H, N***H₂***), 7.41 (t, J=4.7 Hz, 1H, N***H***CH₂CCH).
¹³C NMR (DMSO-*d₆*) δ 13.3 (SCH₂CH₂***C***H₃), 22.7 (SCH₂***C***H₂CH₃), 30.1 (NH***C***H₂CCH), 32.2 (S***C***H₂CH₂CH₃), 73.1 (NHCH₂C***C***H), 81.1 (NHCH₂***C***CH), 120.3 (C-5), 137.9 (C-6), 151.8 (C-4), 155.1 (C-2).

### 6-Chloro-9-(prop-2-yn-1-yl)-2-(propylthio)-9H-purine (17b)

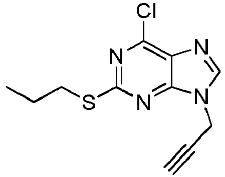

A solution of (**17a**) (258.0 mg, 1 mmol) in acetic acid (2.5 mL) and triethyl orthoformate (2.5 mL, 15 mmol) was heated at a temperature of 130°C under reflux for 2 h. After distillation of the acid acetic and triethyl orthoformate under vacuum, the residue was purified by silica gel column chromatography. Yield: 62%.
Melting point: 68-70°C.
¹H NMR (DMSO-*d₆*) δ 1.01 (t, J=7.4 Hz, 3H, SCH₂CH₂C***H₃***), 1.75 (h, J=7.3 Hz, 2H, SCH₂C***H₂***CH₃), 3.19 (t, J=7.2 Hz, 2H, SC***H₂***CH₂CH₃), 3.57 (d, J=2.5 Hz, 1H, NCH₂CC***H***), 5.13 (d, J=2.5 Hz, 2H, NC***H₂***CCH), 8.58 (s, 1H, 8-***H***).
¹³C NMR (DMSO-*d₆*) δ 13.2 (SCH₂CH₂***C***H₃), 22.0 (SCH₂***C***H₂CH₃), 32.7 (S***C***H₂CH₂CH₃), 33.1 (N***C***H₂CCH), 76.6 (NCH₂C***C***H), 77.2 (NCH₂***C***CH), 127.9 (C-5), 145.5 (C-8), 149.1 (C-6), 152.3 (C-4), 164.4 (C-2).

### N-((1R,2S)-2-(3,4-Difluorophenyl)cyclopropyl)-9-(prop-2-yn-1-yl)-2-(propylthio)-9H-purin-6-amine (17c)

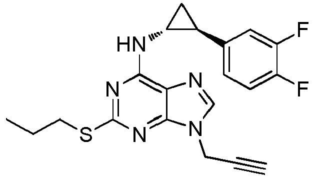

A solution of (**17b**) (134.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 4 h. After distillation of the acetonitrile and triethylamine under vacuum, the residue was purified by silica gel column chromatography.
Yield: 86%.
Melting point: 72-74°C.
¹H NMR (CDCl₃) δ 0.94 (t, J=7.4 Hz, 3H, SCH₂CH₂C***H₃***), 1.33 (m, 2H, NHCH(C***H₂***)CHPh), 1.65 (h, J=7.3 Hz, 2H, SCH₂C***H₂***CH₃), 2.09 (m, 1H, NHCH(CH₂)C***H***Ph), 2.50 (t, J=2.6 Hz, 1H, NCH₂CC***H***), 3.03 (m, 2H, SC***H₂***CH₂CH₃), 3.12 (bs, 1H, NHC***H***(CH₂)CHPh), 4.90 (d, J=2.6 Hz, 2H, NC***H₂***CCH), 6.00 (bs, 1H, N***H***), 6.98 (m, 1H, 6'-H), 7.08 (m, 2H, 2'-H/5'-H), 7.84 (s, 1H, 8-***H***).
¹³C NMR (CDCl₃) δ 13.4 (SCH₂CH₂***C***H₃), 16.1 (NHCH(***C***H₂)CHPh), 22.8 (SCH₂***C***H₂CH₃), 25.2 (NHCH(CH₂)***C***HPh), 32.8 (N***C***H₂CCH), 33.2 (S***C***H₂CH₂CH₃), 33.3 (NH***C***H(CH₂)CHPh), 74.9 (NCH₂C***C***H), 76.1 (NCH₂***C***CH), 115.6 (d, J=17 Hz, C-2'), 116.9 (d, J=17 Hz, C-5'), 117.3 (C-5), 122.6 (C-6'), 137.8 (C-1'), 138.1 (C-8),147.9-149.9 (dd, 246 Hz/13 Hz, C-4'), 149.2-151.2 (dd, 247 Hz/13 Hz, C-3'), 149.9 (C-4), 154.6 (C-6), 166.0 (C-2).

### N-((1R,2S)-2-(3,4-Difluorophenyl)cyclopropyl)-9-(prop-2-yn-1-yl)-2-(propylthio)-9H-purin-6-amine hydrochloride (17c.HCl)

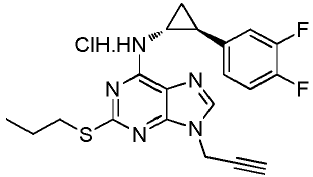

To a solution of (**17c**) (0.5 mmol) in diethyl ether (5 mL) was added dropwise a saturated solution of HCl in diethyl ether. The resulting precipitate of the title compound was collected by filtration, wached with diethyl ether and dried.
Yield: 99%.
Melting point: 178-180°C.

### Example 18: synthesis of N-((1R,2S)-2-(3,4-difluorophenyl)cyclopropyl)-2-(propylthio)-9-(2,2,2-trifluoroethyl)-9H-purin-6-amine (18c)

### 6-Chloro-2-(propylthio)-N⁴-(2,2,2-trifluoroethyl)pyrimidine-4,5-diamine (18a)

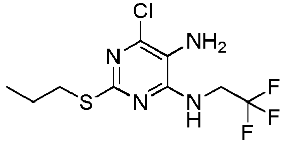

44,6-Dichloro-2-(propylthio)pyrimidin-5-amine (0.5 g, 2.1 mmol) was dissolved in methanol (2 mL) and supplemented with 2,2,2-trifluoroethanamine (625.0 mg, 6.3 mmol). The reaction mixture was introduced in a sealed vessel and heated at 100°C for 24 h. After concentration of the reaction mixture to dryness under vacuum, the residue was purified by silica gel column chromatography.
Yield: 76%.
Melting point: 107-109°C.
¹H NMR (DMSO-*d₆*) δ 0.95 (t, J=7.4 Hz, 3H, SCH₂CH₂C***H₃***), 1.63 (h, J=7.3 Hz, 2H, SCH₂C***H₂***CH₃), 2.95 (t, J=7.2 Hz, 2H, SC***H₂***CH₂CH₃), 4.29 (m, 2H, NHC***H₂***CF₃), 4.95 (s, 2H, N***H₂***), 7.53 (s, 1H, N***H***CH₂CF₃).
¹³C NMR (DMSO-*d₆*) δ 13.2 (SCH₂CH₂***C***H₃), 22.6 (SCH₂***C***H₂CH₃), 32.1 (S***C***H₂CH₂CH₃), 41.2 (q, J=33 Hz, NH***C***H₂CF₃), 120.5 (C-5), 124.3 (m, NHCH₂***C***F₃), 138.8 (C-6), 151.9 (C-4), 154.8 (C-2).

### 6-Chloro-2-(propylthio)-9-(2,2,2-trifluoroethyl)-9H-purine (18b)

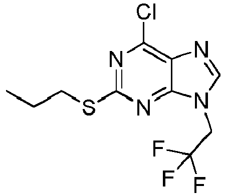

A solution of (**18a**) (301.0 mg, 1 mmol) in acetic acid (2.5 mL) and triethyl orthoformate (2.5 mL, 15 mmol) was heated at a temperature of 130°C under reflux for 2 h. After distillation of the acid acetic and triethyl orthoformate under vacuum, the residue was purified by silica gel column chromatography. Yield: 48%.
Melting point: 140-143°C.
¹H NMR (DMSO-*d₆*) δ 1.00 (t, J=7.4 Hz, 3H, SCH₂CH₂C***H₃***), 1.73 (h, J=7.3 Hz, 2H, SCH₂C***H₂***CH₃), 3.19 (t, J=7.2 Hz, 2H, SC***H₂***CH₂CH₃), 5.27 (q, J=9.2 Hz, 2H, NC***H₂***CF₃), 8.60 (s, 1H, 8-***H***).
¹³C NMR (DMSO-*d₆*) δ 13.1 (SCH₂CH₂***C***H₃), 22.0 (SCH₂***C***H₂CH₃), 32.6 (S***C***H₂CH₂CH₃), 43.9 (q, J=35 Hz, N***C***H₂CF₃), 123.4 (q, J=280 Hz, NCH₂***C***F₃), 127.6 (C-5), 146.2 (C-8), 149.6 (C-6), 152.9 (C-4), 165.2 (C-2).

### N-((1R,2S)-2-(3,4-Difluorophenyl)cyclopropyl)-2-(propylthio)-9-(2,2,2-trifluoroethyl)-9H-purin-6-amine (18c)

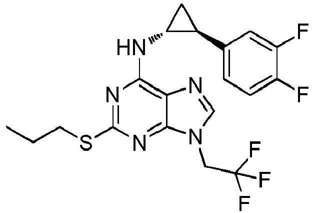

A solution of (**18b**) (156.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 89%.
Melting point: 102-104°C.
¹H NMR (CDCl₃) δ 0.95 (t, J=7.4 Hz, 3H, SCH₂CH₂C***H₃***), 1.34 (m, 2H, NHCH(C***H₂***)CHPh), 1.67 (h, J=7.3 Hz, 2H, SCH₂C***H₂***CH₃), 2.11 (m, 1H, NHCH(CH₂)C***H***Ph), 3.03 (m, 2H, SC***H₂***CH₂CH₃), 3.10 (bs, 1H, NHC***H***(CH₂)CHPh), 4.73 (qd, J=8.5 Hz/3.1 Hz, 2H, NC***H₂***CF₃), 6.06 (bs, 1H, N***H***), 6.99 (m, 1H, 6'-H), 7.09 (m, 2H, 2'-H/5'-H), 7.70 (s, 1H, 8-***H***).
¹³C NMR (CDCl₃) δ 13.4 (SCH₂CH₂***C***H₃), 15.9 (NHCH(***C***H₂)CHPh), 22.7 (SCH₂***C***H₂CH₃), 25.2 (NHCH(CH₂)***C***HPh), 33.2 (S***C***H₂CH₂CH₃), 33.3 (NH***C***H(CH₂)CHPh), 44.0 (q, J=36 Hz, N***C***H₂CF₃), 115.7 (d, J=17 Hz, C-2'), 116.8 (C-5), 117.0 (d, J=17 Hz, C-5'), 122.7 (C-6'), 122.8 (q, J=279 Hz, CF₃), 137.7 (C-1'), 138.2 (C-8), 148.0-150.0 (dd, 246 Hz/13 Hz, C-4'), 149.2-151.2 (dd, 247 Hz/13 Hz, C-3'), 150.6 (C-4), 154.7 (C-6), 166.8 (C-2).

### Example 19: synthesis of (1S,2R,3S,4R)-4-(6-(((1R,2S)-2-(3,4-difluorophenyl)cyclopropyl)amino)-2-(propylthio)-9H-purin-9-yl)cyclopentane-1,2,3-triol (19d)

### (3aR,4S,6R,6aS)-6-((5-Amino-6-chloro-2-(propylthio)pyrimidin-4-yl)amino)-2,2-dimethyltetrahydro-3aH-cyclopenta[d][1,3]dioxol-4-ol (19a)

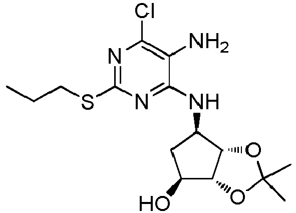

4,6-Dichloro-2-(propylthio)pyrimidin-5-amine (0.5 g, 2.1 mmol) was dissolved in methanol (2 mL) and supplemented with (3a*R*,4*S*,6*R*,6a*S*)-6-amino-2,2-dimethyltetrahydro-3a*H*-cyclopenta[*d*][1,3]dioxol-4-ol (1.1 g, 6.3 mmol). The reaction mixture was introduced in a sealed vessel and heated at 100°C for 12 h. After concentration of the reaction mixture to dryness under vacuum, the residue was purified by silica gel column chromatography.
Yield: 90%.
Melting point: ND.
¹H NMR (DMSO-*d₆*) δ 0.96 (t, J=7.4 Hz, 3H, SCH₂CH₂C***H₃***), 1.21 (s, 3H, C(C***H₃***)*₂*), 1.36 (s, 3H, C(C***H₃***)*₂*), 1.64 (h, J=7.4 Hz, 2H, SCH₂C***H₂***CH₃), 1.71 (m, 1H, 5'-***H***), 2.22 (m, 1H, 5'-***H***), 2.98 (t, J=7.2 Hz, 2H, SC***H₂***CH₂CH₃), 4.06 (bs, 1H, 4'-***H***), 4.26 (bs, 1H, 6'-***H***), 4.41 (d, J=5.9 Hz, 1H, 3a'-***H***), 4.51 (d, J=6.0 Hz, 1H, 6a'-***H***), 4.70 (s, 2H, N***H₂***), 5.27 (d, J=3.1 Hz, 1H, O***H***), 6.63 (d, J=7.1 Hz, 1H, N***H***).
¹³C NMR (DMSO-*d₆*) δ 13.3 (SCH₂CH₂***C***H₃), 22.9 (SCH₂***C***H₂CH₃), 24.1 (C(***C***H₃)*₂*), 26.5 (C(***C***H₃)*₂*), 32.1 (S***C***H₂CH₂CH₃), 35.9 (C-5'), 57.2 (C-6'), 75.3 (C-4'), 84.5 (C-6a'), 85.7 (3a'), 109.7 (***C***(CH₃)₂), 119.7 (C-5), 136.6 (C-6), 152.4 (C-4), 155.9 (C-2).

### (3aR,4S,6R,6aS)-6-(6-Chloro-2-(propylthio)-9H-purin-9-yl)-2,2-dimethyltetrahydro-3aH-cyclopenta[d][1,3]dioxol-4-ol (19b)

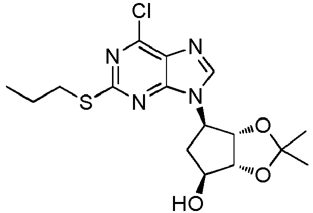

A solution of (**19a**) (375.0 mg, 1 mmol) in acetic acid (2.5 mL) and triethyl orthoformate (2.5 mL, 15 mmol) was heated at a temperature of 130°C under reflux for 10 h. After distillation of the acid acetic and triethyl orthoformate under vacuum, the residue was purified by silica gel column chromatography. Yield: 23%.
Melting point: ND.
¹H NMR (DMSO-*d₆*) δ 1.02 (t, J=7.4 Hz, 3H, SCH₂CH₂C***H₃***), 1.25 (s, 3H, C(C***H₃***)*₂*), 1.45 (s, 3H, C(C***H₃***)*₂*), 1.75 (h, J=7.2 Hz, 2H, SCH₂C***H₂***CH₃), 2.25 (m, 1H, 5'-***H***), 2.51 (m, 1H, 5'-***H***), 3.16 (t, J=7.2 Hz, 2H, SC***H₂***CH₂CH₃), 4.18 (bs, 1H, 4'-***H***), 4.56 (d, J=6.2 Hz, 1H, 3a'-***H***), 4.86 (m, 1H, 6'-***H***), 5.04 (dd, J=6.1 Hz/1.9 Hz, 1H, 6a'-***H***), 5.47 (bs, 1H, O***H***), 8.59 (s, 1H, 8-***H***).
¹³C NMR (DMSO-*d₆*) δ 13.3 (SCH₂CH₂***C***H₃), 22.2 (SCH₂***C***H₂CH₃), 24.3 (C(***C***H₃)*₂*), 26.6 (C(***C***H₃)*₂*), 32.7 (S***C***H₂CH₂CH₃), 36.4 (C-5'), 60.4 (C-6'), 74.6 (C-4'), 83.9 (C-6a'), 86.1 (C-3a'), 110.9 (***C***(CH₃)₂), 128.1 (C-5), 146.0 (C-8), 148.9 (C-6), 152.7 (C-4), 163.9 (C-2).

### (3aR,4S,6R,6aS)-6-(6-(((1R,2S)-2-(3,4-Difluorophenyl)cyclopropyl)amino)-2-(propylthio)-9H-purin-9-yl)-2,2-dimethyltetrahydro-3aH-cyclopenta[d][1,3]dioxol-4-ol (19c)

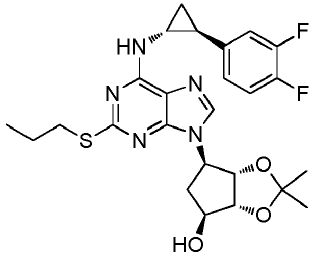

A solution of (**19b**) (193.0 mg, 0.5 mmol) in acetonitrile (2.5 mL) was supplemented with (1*R*,2*S*)-2-(3,4-difluorophenyl)cyclopropanamine (93.0 mg, 0.55 mmol) and triethylamine (0.13 mL) and then heated at 90°C under reflux for 1 h. After distillation of the acetonitrile and triethylamine under vacuum, the residue was purified by silica gel column chromatography.
Yield: 91%.
Melting point: ND.
¹H NMR (CDCl₃) δ 0.90 (t, J=7.4 Hz, 3H, SCH₂CH₂C***H₃***), 1.32 (s, 3H, C(***C***H₃)*₂*), 1.34 (m, 2H, NHCH(C***H₂***)CHPh), 1.51 (s, 3H, C(***C***H₃)₂), 1.61 (h, J=7.3 Hz, 2H, SCH₂C***H₂***CH₃), 2.09 (m, 1H, NHCH(CH₂)C***H***Ph), 2.13 (m, 1H, 5"-***H***), 2.96 (m, 3H, 5"-***H***/SC***H₂***CH₂CH₃), 3.13 (bs, 1H, NHC***H***(CH₂)CHPh), 4.42 (m, 1H, 4"-***H***), 4.74 (m, 1H, 6"-***H***), 4.79 (d, J=5.3 Hz, 1H, 3a"-***H***), 4.98 (d, J=5.3 Hz, 1H, 6a"-***H***), 5.99 (bs, 1H, O***H***), 6.03 (bs, 1H, N***H***), 6.93 (m, 1H, 6'-H), 7.05 (m, 2H, 2'-H/5'-H), 7.67 (s, 1H, 8-***H***).
¹³C NMR (CDCl₃) δ 13.3 (SCH₂CH₂***C***H₃), 16.2 (NHCH(***C***H₂)CHPh), 22.6 (SCH₂***C***H₂CH₃), 24.5 (C(***C***H₃)*₂*), 25.2 (NHCH(CH₂)***C***HPh), 27.1 (C(***C***H₃)*₂*), 33.2 (S***C***H₂CH₂CH₃), 33.3 (NH***C***H(CH₂)CHPh), 38.8 (C-5"), 64.0 (C-6"), 76.0 (C-4"), 86.2 (C-6a"), 88.0 (C-3a"), 111.4 (***C***(CH₃)₂), 115.3 (d, J=17 Hz, C-2'), 117.0 (d, J=17 Hz, C-5'), 118.3 (C-5), 122.4 (C-6'), 137.7 (C-1'), 139.8 (C-8), 148.0-150.0 (dd, 246 Hz/13 Hz, C-4'), 149.3-151.3 (dd, 247 Hz/13 Hz, C-3'), 150.5 (C-4), 154.7 (C-6), 165.7 (C-2).

### (1S,2R,3S,4R)-4-(6-(((1R,2S)-2-(3,4-Difluorophenyl)cyclopropyl)amino)-2-(propylthio)-9H-purin-9-yl)cyclopentane-1,2,3-triol (19d)

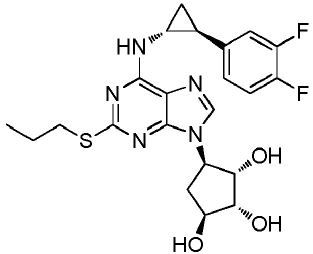

A solution of (**19c**) (0.5 mmol) in methanol (2 mL) and 12N HCl (1 mL) was stirred at room temperature for 2 h. After distillation of the solvents under vacuum, the residue was purified by silica gel column chromatography.
Yield: 76%.
Melting point: 92-94°C.
¹H NMR (CDCl₃) δ 0.87 (t, J=7.3 Hz, 3H, SCH₂CH₂C***H₃***), 1.34 (m, 2H, NHCH(C***H₂***)CHPh), 1.59 (h, J=7.3 Hz, 2H, SCH₂C***H₂***CH₃), 2.10 (m, 1H, NHCH(CH₂)C***H***Ph), 2.14 (m, 1H, 5"-***H***), 2.71 (bs, 1H, 3"-O***H***), 2.83 (m, 1H, SC***H₂***CH₂CH₃), 2.98 (m, 2H, 5"-***H***/SC***H₂***CH₂CH₃), 3.12 (bs, 1H, NHC***H***(CH₂)CHPh), 4.14 (m, 1H, 3"-***H***), 4.27 (m, 1H, 1"-***H***), 4.47 (bs, 1H, 2"-O***H***), 4.56 (m, 1H, 4"-***H***), 4.76 (m, 1H, 2"-***H***), 4.98 (bs, 1H, 1"-O***H***), 6.16 (bs, 1H, N***H***), 6.93 (m, 1H, 6'-H), 7.04 (m, 2H, 2'-H/5'-***H***), 7.59 (s, 1H, 8-***H***).
¹³C NMR (CDCl₃) δ 13.2 (SCH₂CH₂***C***H₃), 16.2 (NHCH(***C***H₂)CHPh), 22.5 (SCH₂***C***H₂CH₃), 25.2
(NHCH(CH₂)***C***HPh), 33.1 (S***C***H₂CH₂CH₃), 33.3 (NH***C***H(CH₂)CHPh), 35.8 (C-5"), 61.5 (C-4"), 74.8 (C-1"), 76.9 (C-2"), 78.0 (C-3"), 115.3 (d, J=17 Hz, C-2'), 117.0 (d, J=17 Hz, C-5'), 118.3 (C-5), 122.3 (C-6'), 137.8 (C-1'), 139.0 (C-8), 148.0-150.0 (dd, 246 Hz/13 Hz, C-4'), 149.3-151.3 (dd, 247 Hz/13 Hz, C-3'), 149.3 (C-4), 154.6 (C-6), 165.8 (C-2).

### 2.Examples of pyrimidines derivatives for use in prevention and treatment of bacterial infection

### Example 1: Antibacterial effects of molecules 2329, 2348, 2412, 2452, and 2461 on S. epidermidis: determination of Minimal Inhibitory Concentration (MIC).

Molecules 2329, 2348, 2412, 2452, and 2461 correspond respectively to the following formulations: 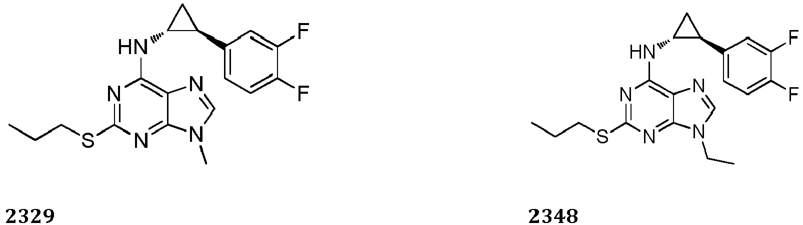
2329 is *N*-((1*R*,2*S*)-2-(3,4-difluorophenyl)cyclopropyl)-9-methyl-2-(propylthio)-9*H-*purin-6-amine (also called 1c above)
2348 is *N*-((1*R*,2*S*)-2-(3,4-difluorophenyl)cyclopropyl)-9-ethyl-2-(propylthio)-9*H*-purin-6-amine (also called 3c above)

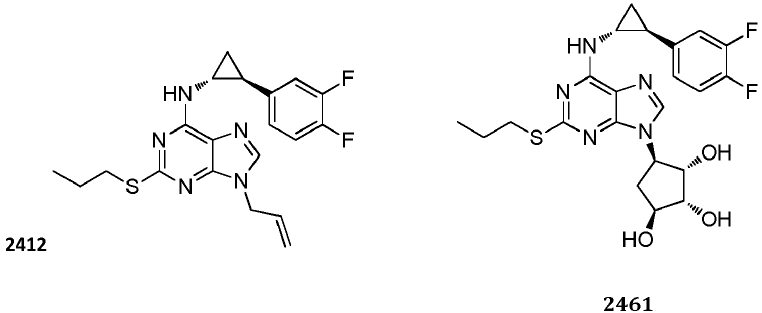

2412 is 9-allyl-*N*-((1*R*,2*S*)-2-(3,4-difluorophenyl)cyclopropyl)-2-(propylthio)-9*H*-purin-6-amine (also called 15c)
2461 is (1*S*,2*R*,3*S*,4*R*)-4-(6-(((1*R*,2*S*)-2-(3,4-difluorophenyl)cyclopropyl)amino)-2-(propylthio)-9*H*-purin-9-yl)cyclopentane-1,2,3-triol (also called 19d). 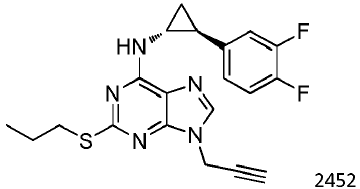
2452 is *N*-((1*R*,2*S*)-2-(3,4-difluorophenyl)cyclopropyl)-9-(prop-2-yn-1-yl)-2-(propylthio)-9*H*-purin-6-amine hydrochloride (also called 17c).

The Minimal Inhibitory Concentration (MIC) of molecules 2329, 2348, 2412, 2452, and 2461 was determined on *Staphylococcus epidermidis* (ATCC 35984, also known as RP62A) according to EUCAST (European Committee on Antimicrobial Susceptibility Testing) recommendations.

Briefly, a single colony grown on a Tryptic Soy Agar (TSA) plate was resuspended and cultured in Tryptic Soy Broth (TSB) overnight (O/N) in aerobic conditions (37°C with 220rpm shaking), next day a 1:50 inoculum in Mueller-Hinton broth (MHB) was incubated in aerobic conditions for 3hr and an inoculum of 1:100 dilution, corresponding to 3x10⁵ CFU/ml, was incubated in presence or absence of different concentrations of the molecules in 1% DMSO (vehicle). After O/N growth the OD of each culture was measured at 600nm in a spectrophotometer (OD₆₀₀). The MIC represents the concentration at which there is no visible growth of bacteria, *i.e.* ΔOD at 600nm equal to zero (blank is the medium alone).

The MIC for molecules 2329, 2348, and 2461 against *S.epidermidis* is equal to 20 µM, while it is above 50 µM for molecules 2412 and 2452.

### Example 2: Antibacterial effects of molecules 2329, 2348, 2412, 2452, and 2461 on S. aureus: determination of Minimal Inhibitory Concentration (MIC).

Further experiments are conducted using different strains of *S. aureus*, as clinically relevant Gram-positive bacterial strains: *S. aureus* ATCC 25904, methilcillin-resistant *S. aureus* (MRSA) ATCC BAA-1556, Glycopeptide intermediate-resistant (GISA) *S. aureus* Mu-50 (ATCC 700695) in order to determine the Minimal Inhibitory Concentration (MIC) which is the minimal concentration required to prevent bacterial growth. The bioluminescent strain *S. aureus* Xen29 was also used. This strain is derived from the parental strain *S. aureus* 12600, a pleural fluid isolate, which is also designated as NCTC8532. *S. aureus* Xen29 possesses a stable copy of the modified Photorhabdus luminescens luxABCDE operon at a single integration site on the bacterial chromosome.

A single colony selected from the different strains of *S. aureus* is resuspended and cultured in the appropriate medium overnight (O/N) in aerobic conditions (37°C with 220rpm shaking), next day a 1:100 inoculum in Mueller-Hinton broth (MHB) is incubated in aerobic conditions for 3hr (OD=0,08-0,1) and an inoculum of 1:300 dilution, corresponding to 3x10⁵ CFU/ml, is incubated in presence or absence of different concentrations of the tested molecules in 1% DMSO. After O/N growth the OD of each culture was measured at 600nm in a spectrophotometer (OD₆₀₀). The MIC represents the concentration at which there is no visible growth of bacteria, *i.e.* ΔOD at 600nm equal to zero (blank is the medium alone). MIC for 2329, 2348, 2412, 2452, and 2461 against *S. aureus* ATCC 25904 and Xen29 are equal to 20-25 µM). Molecule 2329 is the most potent against methilcillin-resistant *S. aureus* (MRSA) ATCC BAA-1556, with a MIC equal to 20 µM), while molecules 2348 and 2461 had a MIC of 25 µM), and the MIC for molecules 2412 and 2452 is comprised between 2-25 and 50 µM. The MIC of molecule 2329 against Glycopeptide intermediate-resistant (GISA) *S. aureus* Mu-50 (ATCC 700695) is in between 25 and 30 µM, while it is above 50 µM for molecules 2348, 2412, and 2452.

### Example 3: Antibacterial effects of molecules 2329, 2348, 2412, 2452, and 2461 on E. faecalis: determination of Minimal Inhibitory Concentration (MIC)

Further experiments were conducted using the clinically relevant Gram-positive bacterial strain of vancomycin-resistant (VRE) *E. faecalis* ATCC BAA-2365, in order to determine the Minimal Inhibitory Concentration (MIC) which is the minimal concentration required to prevent bacterial growth.

A single colony selected from *E. faecalis* VRE is resuspended and cultured in the appropriate medium overnight (O/N) in aerobic conditions (37°C with 220rpm shaking), next day a 1:100 inoculum in Mueller-Hinton broth (MHB) is incubated in aerobic conditions for 3hr (OD=0,08-0,1) and an inoculum of 1:300 dilution, corresponding to 3x10⁵ CFU/ml, is incubated in presence or absence of different concentrations of the tested molecules in 1% DMSO. After O/N growth the OD of each culture is measured at 600nm in a spectrophotometer (OD₆₀₀). The MIC represents the concentration at which there is no visible growth of bacteria, *i.e.* ΔOD at 600nm equal to zero (blank is the medium alone). MIC for molecules 2329 and 2461 against *E. faecalis* vancomycin-resistant (VRE) ATCC BAA-2365 was equal to 25 µM, while it is above 50 µM for molecules 2348, 2412 and 2452.

The results of all experiments are illustrated in Table 1.

**Table 1. MIC: minimal inhibitory concentration; MRSA: methilcillin-resistant S.aureus; GISA: Glycopeptide intermediate-resistant S.aureus; VRE: vancomycin-resistant E. faecalis; nd: not determined.**

| Strains | S.aureus | | | | S.epidermidis | E.faecalis |
|---|---|---|---|---|---|---|
| Molecules | ATCC25904 | Xen29 | MRSA | GISA | RP62 | VRE |
| 2329 | 20-25µM | 20-25µM | 20µM | 25-30µM | 20µM | 25µM |
| 2348 | 20-25µM | 20-25µM | 25µM | >50µM | 20µM | >50µM |
| 2412 | 20-25µM | 20-25µM | 25-50µM | >50µM | >50µM | >50µM |
| 2452 | 20-25µM | 20-25µM | 25-50µM | >50µM | >50µM | >50µM |
| 2461 | 20-25µM | 20-25µM | 25µM | nd | 20µM | 25µM |

### Exemple 4: use of molecules 2329, 2348 as inhibitors of S. aureus biofilm formation.

Bioluminescent *S. aureus* (Xen29, Perkin Elmer) is grown overnight in TSB medium, before being diluted 100 fold in fresh TSB, and incubated aerobically at 37°C until bacteria culture reached an OD₆₀₀ of 0.6 (corresponding to approximately 1-3x10⁸ CFU/ml). Bacteria cultures are then diluted to 1x10⁴ CFU/ml in fresh TSB. 600 µl aliquots of diluted bacteria suspensions are distributed in each well of a 48-well plate. Bacteria are allowed to adhere for 3 hours under static conditions at 37°C. After removing media, wells are rinsed 2 times with PBS to eliminate planktonic bacteria and re-filled with TSB supplemented with 0.5 % glucose

Molecules 2329 or 2348 are then added at 10µM final concentration. Biofilm formation is imaged every 60 min, for 13 hours, using a IVIS camera system (Xenogen Corp.) Total photon emission from each well is then quantified using the Living Image software package (Xenogen Corp.).

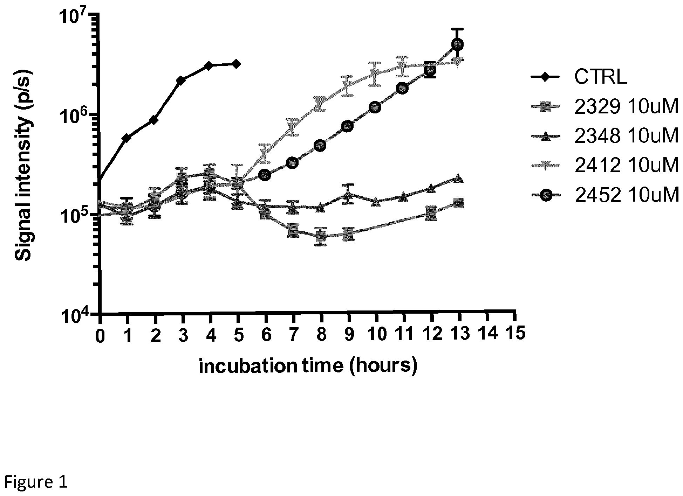

In figure 1, biofilm formation is represented as the intensity of photon signal per surface unit. Molecules 2329 and 2348 fully prevented biofilm formation when used at a dose of 10µM.

### Exemple 5: use of molecules 2412 and 2452 to delay S. aureus biofilm formation.

Bioluminescent *S. aureus* (Xen29, Perkin Elmer) is grown overnight in TSB medium, before being diluted 100 fold in fresh TSB, and incubated aerobically at 37°C until bacteria culture reached an OD₆₀₀ of 0.6 (corresponding to approximately 1-3x10⁸ CFU/ml). Bacteria cultures are then diluted to 1x10⁴ CFU/ml in fresh TSB. 600 µl aliquots of diluted bacteria suspensions were distributed in each well of a 48-well plate. Bacteria are allowed to adhere for 3 hours under static conditions at 37°C. After removing media, wells are rinsed 2 times with PBS to eliminate planktonic bacteria and re-filled with TSB supplemented with 0.5 % glucose

Molecules 2412 or 2452 are then added at 10µM final concentration. Biofilm formation is imaged every 60 min, for 13 hours, using a IVIS camera system (Xenogen Corp.) Total photon emission from each well is then quantified using the Living Image software package (Xenogen Corp.).
In figure 1, biofilm formation is represented as the intensity of photon signal per surface unit. Molecules 2412 and 2452 are able to delay biofilm formation when used at a dose of 10µM as compared to vehicle control (DMSO 1%).

### Example 6: Molecule 2329 destroys S. epidermidis mature biofilm

In another experiment we let adhere 0.5x10⁸ CFU/ml *S. epidermidis* cells for 4hr and let the biofilm form for additional 24hr in presence of 0.25% glucose, at this point we treated the biofilm with different concentrations of molecule 2329 for 24h in TSB with 0.25% glucose and determined the biofilm biomass using the crystal violet staining.

For biofilm analysis, we first washed the biofilm 3 times with NaCl 0,9% to eliminate all planktonic bacteria, before incubation with a staining Crystal Violet 1% solution in distilled H₂0 (dH₂0).

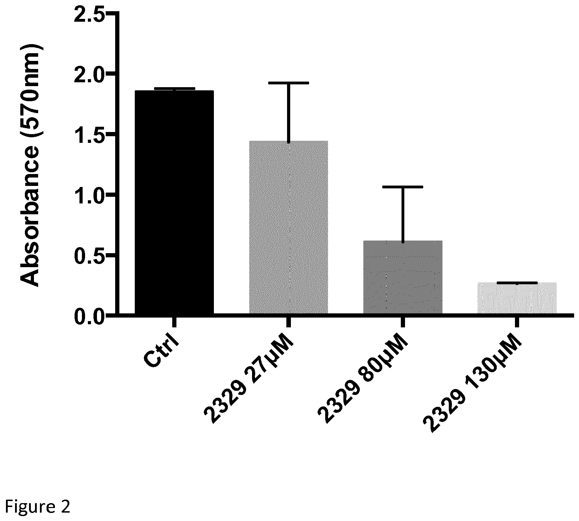

Wells are washed 3 times with dH₂0 to eliminate unbound crystal violet. 400µl Acetic Acid 10% is then added and incubated at RT for 10min. Absorbance is measured in triplicate at 570nm, reflecting total biomass of the biofilm. Figure 2 shows that 2329 80µM could destroy the *S. epidermidis* 24-hour mature biofilm.

## Claims

1. Pyrimidine derivatives represented by formula (I) 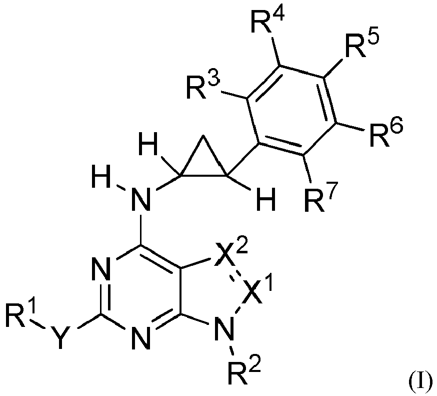 or an acceptable salt or prodrug thereof;
wherein:
X¹ and X² are independently N, CH, CR⁸ wherein R⁸ is C₁₋₆ alkyl, C₂₋₆ alkenyl or C₂₋₆ alkynyl; with the exception that if X¹ or X² is equal to N, then X¹ and X² are different; -Y- is -O- or -S-;
R¹ and R² are independently C₁₋₆-alkyl, C₂₋₆-alkenyl, C₂₋₆-alkynyl, C₃₋₆-cycloalkyl, aryl, aryl-C₁₋₆-alkyl wherein the alkyl moiety is optionally mono or polysubstituted with OH or an halogen and the aryl moiety is optionally mono or polysubstituted with an halogen, -C₁₋₆alkyl, -C₁₋₆alkoxy, -OH, -NO₂, -CN, -NH₂, -NHR⁸, -N(R⁸)₂ -COOH, -COOR⁸,-CONH₂, -CONHR⁸, -CON(R⁸)₂, -SO₂NH₂, -SO₂NHR⁸, or -SO₂N(R⁸)₂;
R³, R⁴, R⁵, R⁶ and R⁷ are independently H, an halogen, a C₁₋₆alkyl, C₁₋₆alkoxy, -OH,,-NO₂,, -CN, -NH₂, -NHR⁸, -N(R⁸)₂ -COOH, -COOR⁸, -CONH₂, -CONHR⁸, -CON(R⁸)₂,-SO₂NH₂, -SO₂NHR⁸, or -SO₂N(R⁸)₂;

2. The Pyrimidine derivative according to claim 1 wherein X¹ is CH or CR⁸ and X² is N.

3. The Pyrymidine derivatives according to claim 2 selected from:
*N*-((1*R*,2*S*)-2-(3,4-difluorophenyl)cyclopropyl)-9-methyl-2-(propylthio)-9*H*-purin-6-amine (1c);
9-methyl-*N*-((1*R*,2*S*)-2-phenylcyclopropyl)-2-(propylthio)-9*H*-purin-6-amine (2c);
*N*-((1*R*,2*S*)-2-(3,4-difluorophenyl)cyclopropyl)-9-ethyl-2-(propylthio)-9*H*-purin-6-amine (3c);
*N*-((1*R*,2*S*)-2-(3,4-difluorophenyl)cyclopropyl)-9-propyl-2-(propylthio)-9*H*-purin-6-amine (4c);
*N*-((1*R*,2*S*)-2-(3,4-difluorophenyl)cyclopropyl)-9-isopropyl-2-(propylthio)-9*H*-purin-6-amine (5c);
9-cyclopropyl-*N*-((1*R*,2*S*)-2-(3,4-difluorophenyl)cyclopropyl)-2-(propylthio)-9*H*-purin-6-amine (6c);
9-butyl-*N*-((1*R,*2*S*)-2-(3,4-difluorophenyl)cyclopropyl)-2-(propylthio)-9*H*-purin-6-amine (7c);
9-(*sec*-butyl)-*N*-((1*R*,2*S*)-2-(3,4-difluorophenyl)cyclopropyl)-2-(propylthio)-9*H*-purin-6-amine (8c);
9-(*tert*-butyl)-*N*-((1*R*,2*S*)-2-(3,4-difluorophenyl)cyclopropyl)-2-(propylthio)-9*H*-purin-6-amine (9c);
9-cyclobutyl-*N*-((1*R*,2*S*)-2-(3,4-difluorophenyl)cyclopropyl)-2-(propylthio)-9*H*-purin-6-amine (10c);
N-((1*R*,2*S*)-2-(3,4-difluorophenyl)cyclopropyl)-9-pentyl-2-(propylthio)-9*H*-purin-6-amine (11c);
9-cyclopentyl-*N*-((1*R*,2*S*)-2-(3,4-difluorophenyl)cyclopropyl)-2-(propylthio)-9*H*-purin-6-amine (12c);
*N*-((1*R*,2*S*)-2-(3,4-difluorophenyl)cyclopropyl)-9-hexyl-2-(propylthio)-9*H*-purin-6-amine (13c);
9-cyclohexyl-*N*-((1*R*,2*S*)-2-(3,4-difluorophenyl)cyclopropyl)-2-(propylthio)-9*H*-purin-6-amine (14c);
9-allyl-*N*-((1*R*,2*S*)-2-(3,4-difluorophenyl)cyclopropyl)-2-(propylthio)-9*H*-purin-6-amine (15c);
2-(6-(((1*R*,2*S*)-2-(3,4-difluorophenyl)cyclopropyl)amino)-2-(propylthio)-9*H*-purin-9-yl)ethanol (16c);
*N*-((1*R*,2*S*)-2-(3,4-difluorophenyl)cyclopropyl)-9-(prop-2-yn-1-yl)-2-(propylthio)-9*H-*purin-6-amine hydrochloride (17c.HCl);
*N*-((1*R*,2*S*)-2-(3,4-difluorophenyl)cyclopropyl)-2-(propylthio)-9-(2,2,2-trifluoroethyl)-9*H*-purin-6-amine (18c);
(1*S*,2*R*,3*S*,4R)-4-(6-(((1*R*,2*S*)-2-(3,4-difluorophenyl)cyclopropyl)amino)-2-(propylthio)-9*H*-purin-9-yl)cyclopentane-1,2,3-triol (19d);
or an acceptable salt or prodrug thereof.

4. The Pyrymidine derivatives according to claim 2 or 3 selected from:
*N*-((1*R*,2*S*)-2-(3,4-difluorophenyl)cyclopropyl)-9-methyl-2-(propylthio)-9*H*-purin-6-amine;
9-allyl-*N*-((1*R*,2*S*)-2-(3,4-difluorophenyl)cyclopropyl)-2-(propylthio)-9*H*-purin-6-amine; *N*-((1*R*,2*S*)-2-(3,4-difluorophenyl)cyclopropyl)-9-(prop-2-yn-1-yl)-2-(propylthio)-9*H-*purin-6-amine;
(1*S*,2*R*,3*S*,4*R*)-4-(6-(((1*R*,2*S*)-2-(3,4-difluorophenyl)cyclopropyl)amino)-2-(propylthio)-9*H*-purin-9-yl)cyclopentane-1,2,3-triol;
*N*-((1*R*,2*S*)-2-(3,4-difluorophenyl)cyclopropyl)-9-(prop-2-yn-1-yl)-2-(propylthio)-9*H-*purin-6-amine hydrochloride;
or an acceptable salt or prodrug thereof.

5. The Pyrimidine derivative according to anyone of claim 1 to 4 which is *N*-((1*R*,2*S*)-2-(3,4-difluorophenyl)cyclopropyl)-9-methyl-2-(propylthio)-9*H*-purin-6-amine.

6. The Pyrimidine derivative according to claim 1 wherein X¹ is N and X² is CH or CR⁸.

7. The Pyrimidine derivative according to claim 1 wherein X¹ and X² are CH or CR⁸.

8. The Pyrimidine derivative according to anyone of claim 1 to 7 wherein R³ and R⁷ are H and R⁴, R⁵ are an halogen, preferably F.

9. The Pyrimidine derivative according to anyone of claim 1 to 8 for use in therapy.

10. The Pyrimidine derivative according to anyone of claim 1 to 9 for use in treatment or prevention of bacterial infection in a host mammal in need of need such treatment or prevention.

11. A pharmaceutical composition comprising a Pyrimidine derivative according to anyone of claim 1 to 8 in combination with pharmaceutically acceptable diluent, adjuvant and/or carrier.

12. A pharmaceutical composition comprising a Pyrimidine derivative according to anyone of claim 1 to 8 for use for use in treatment or prevention of bacterial infection in a host mammal in need of such treatment or prevention.

13. Use of Pyrimidine derivatives according to anyone of claim 1 to 8 as inhibitor of biofilm formation on a surface.

14. A method for killing or controlling bacterial growth in biofilm formation comprising applying on a surface, an effective amount of pyrimidine derivatives according to anyone of claim 1 to 8.

15. The method according to claim 14 wherein the surface belongs to a biomaterial.

16. The method according to claim 15 wherein the biomaterial is a cardiovascular device, preferably a prosthetic heart valve.

17. The method according to claim 15 wherein the biomaterial is a cardiovascular device, preferably a pacemaker.
